# EUROPEAN PATENT APPLICATION

(11) **EP 1 728 512 A1**
(43) Date of publication of application: **06.12.2006**
(21) Application number: 05727021.7
(22) Date of filing: 23.03.2005
(51) Int. Cl.: A61K 31/4439, A61K 9/14, A61K 9/36, A61K 9/62, A61K 47/06, A61K 47/10, A61K 47/12, A61K 47/14, A61K 47/32, A61K 47/38, A61P 1/04, C07D 401/12

(54) **CONTROLLED-LEACHING PREPARATION AND PROCESS FOR PRODUCING THE SAME**

(30) Priority: 26.03.2004 JP 2004093506
(71) Applicant: Eisai R&D Management Co., Ltd., Tokyo 112-8088 (JP)
(72) Inventor: YOSHITAKE, Takashi, c/o Kawashima Industrial Park, Kakamigahara-shi, Gifu 501-6195 (JP); MIZUNO, Mitsuru, c/o Kawashima Industrial Park, Kakamigahara-shi, Gifu 501-6195 (JP); MOROSHIMA, Kenji, c/o Kawashima Industrial Park, Kakamigahara-shi, Gifu 501-6195 (JP); AOKI, Shigeru, c/o Kawashima Industrial Park, Kakamigahara-shi, Gifu 501-6195 (JP)
(74) Representative: HOFFMANN EITLE
(86) International application number: PCT/JP2005/005217
(87) International publication number: WO 2005/092336

(57) **Abstract**

It is an object of the present invention, in the case of a controlled-release pharmaceutical composition, particularly a pulsed-release pharmaceutical composition, containing an acid-unstable physiologically active substance, to provide a pharmaceutical composition having little variation in dissolution lag time and high reliability of dissolution characteristics. The present invention discloses a controlled-release pharmaceutical composition comprising: 1) a core containing an acid-unstable physiologically active substance and a disintegrant; and 2) a release-controlling coating which covers the core, and which contains a water-insoluble polymer, an enteric polymer and a hydrophobic wax.

## Description

### Technical Field

The present invention relates to a controlled-release pharmaceutical composition, and more particularly relates to a pulsed-release pharmaceutical composition, which is one type of the controlled-release pharmaceutical composition, containing a gastric acid secretion inhibitor that is an acid-unstable physiologically active substance.

### Background Art

Hitherto, when preparing an orally administered solid pharmaceutical composition of an acid-unstable physiologically active substance, the pharmaceutical composition has been in general made to be enteric pharmaceutical composition in such a way that the physiologically active substance will dissolve out in the intestines at a neutral to alkaline pH, with decomposition in the stomach being prevented. Moreover, an alkaline additive has been further added as appropriate so as to secure the stability of the acid-unstable physiologically active substance.

A benzimidazole-based compound which has a proton pump inhibitory action and strongly suppresses gastric acid secretion is, for example, well known as an acid-unstable physiologically active substance. Specifically, omeprazole, esomeprazole, lansoprazole, rabeprazole, pantoprazole and so on are used as enteric pharmaceutical compositions, with an alkaline additive being added as required. As compared to a histamine H₂ receptor antagonist, these enteric pharmaceutical compositions have a more powerful and sustained action, and hence are generally administered once a day.

However, depending on the conditions of the patient, there are cases where it is desirable to make the benzimidazole-based compound having proton pump inhibitory action a more sustained release sufficient to maintain the concentration thereof in the blood, thus producing a controlled-release pharmaceutical composition with an excellent therapeutic efficacy of suppressing gastric acid secretion or the like during the night when taken in the morning, i.e. with an improved night-time therapeutic efficacy.

When producing a controlled-release pharmaceutical composition with a longer medical benefit duration, which can be selected in accordance with the symptoms of the patient, it is difficult to obtain sustained release if the core containing the benzimidazole-based compound is coated with only an enteric base. Moreover, if the core containing the benzimidazole-based compound is coated with only a water-insoluble polymer, there may be possibilities that the benzimidazole-based compound decomposes in a gastric acid.

As an another strategy for attaining sustained release, attempts have been reported to make a gradual release the benzimidazole-based compound by forming a matrix with a higher alcohol or a fatty acid ester (see, for example, International Patent Publication Laid-open No. WO 00/74654), but there are concerns that the benzimidazole-based compound may be decomposed by gastric acid in the stomach. Moreover, a sustained-release pharmaceutical composition in which a release-controlling film is provided on the inside of an enteric coating of an omeprazole-containing pharmaceutical composition has been disclosed (see, for example, International Patent Publication Laid-open No. WO 99/32091). However, an acid-unstable physiologically active substance decomposes gradually under acidic or neutral conditions, and hence there are demands for a pulsed-release pharmaceutical composition that enables an acid-unstable physiologically active substance to be released in a pulsed way around the small intestine to the large intestine where the pH is neutral to alkaline, rather than a sustained-release pharmaceutical composition for which the physiologically active substance dissolves out gradually in the gastrointestinal tract and is then prone to decompose.

Here, in the case of a controlled-release pharmaceutical composition, particularly a pulsed-release pharmaceutical composition, it is important to secure the reliability of the dissolution. After being taken, a controlled-release pharmaceutical composition passes through the oral cavity, the esophagus, the stomach, the duodenum, the small intestine, the large intestine and the colon in this order while to some extent maintaining the shape of a tablet, granules, fine granules or the like. The time taken to pass through the alimentary tract varies according to individual differences between people and the type and amount of food eaten, and is said to be 0 to 2 hours, but there is little variation for the small intestine, with the time taken to pass through the small intestine known to generally be approximately 3 hours. However, the pH in the alimentary tract varies from approximately 1 to 8, with individual differences between people being large and control being difficult, and hence in a controlled-release pharmaceutical composition, it is desirable to design the pharmaceutical composition to make variation in dissolution with the pH in the gastrointestinal tract slight. Specifically, the pH in the alimentary tract is said to be approximately 6.8 in the upper part of the small intestine and approximately 7.4 in the large intestine, and if the time from a pharmaceutical composition being taken to pulsed dissolution taking place (lag time) varies greatly due to variation in pH, then it will not be possible to make pulsed-dissolution taking place in the desired time, and hence obtaining a reliable therapeutic efficacy will be difficult. Moreover, there are demands for a pharmaceutical composition for which variation in dissolution lag time within a production lot or between lots is not prone to arise.

Incidentally, a controlled-release pharmaceutical composition in which a core containing an acid-unstable physiologically active substance is coated with a coating containing an enteric polymer and a water-insoluble polymer has been disclosed (see, for example, International Patent Publication Laid-open No. WO 03/043661), and moreover a controlled-release pharmaceutical composition in which a core substance containing a drug and a water-swellable substance is covered with a coating containing an enteric polymer and a water-insoluble polymer has been disclosed (see, for example, Japanese Patent Publication Laid-open No. 2001-55322). However, in the pharmaceutical compositions produced in the prior art, there may be variation in the dissolution lag time of the physiologically active substance, and hence from the above viewpoints, there is need for a pharmaceutical composition having yet less variation in the dissolution lag time and higher reliability of the dissolution characteristics.

### Disclosure of Invention

### Problem to be Solved by the Invention

As described above, in the case of a controlled-release pharmaceutical composition, particularly a pulsed-release pharmaceutical composition, containing an acid-unstable physiologically active substance, there are demands for a pharmaceutical composition having little variation in dissolution lag time and high reliability of dissolution characteristics. That is, there are demands for a controlled-release pharmaceutical composition for which the variation in percentage of dissolution over time and dissolution lag time within a lot or between lots in the same test solution is low, and moreover variation in the percentage of dissolution and the dissolution lag time with various pH in test solutions is low. Furthermore, a disintegrant is often added to the core of a pulsed-release pharmaceutical composition, whereby moisture is absorbed and hence the core swells and thus cracks arise in the pulsed release-controlling coating, thereby the pulsed release effect being impaired. There are thus demands for a pharmaceutical composition for which cracking of the pulsed release-controlling coating does not occur even upon exposure to high-humidity conditions.

### Means for Solving the Problem

In view of the above, as a controlled-release pharmaceutical composition, particularly a pulsed-release pharmaceutical composition, containing an acid-unstable physiologically active substance, the present inventors carried out assiduous studies searching for a controlled-release pharmaceutical composition that has little variation in dissolution lag time. As a result, the present inventors have discovered that this initial object can be attained through the constitution described below, thus arriving at the present invention.

That is, in a first aspect, the present invention provides:
[1] a controlled-release pharmaceutical composition, comprising:1) a core containing an acid-unstable physiologically active substance and a disintegrant; and 2) a release-controlling coating which covers the core, and which contains a water-insoluble polymer, an enteric polymer and a hydrophobic wax,
[2] the controlled-release pharmaceutical composition according to the above [1], wherein the release-controlling coating further comprises a plasticizer,
[3] the controlled-release pharmaceutical composition according to the above [1] or [2], wherein the core further comprises an alkaline additive,
[4] the controlled-release pharmaceutical composition according to any one of the above [1] through [3], further comprising an inert intermediate coating between the core and the release-controlling coating,
[5] the controlled-release pharmaceutical composition according to any one of the above [1] through [4], wherein the controlled-release pharmaceutical composition is a pulsed-release pharmaceutical composition,
[6] the controlled-release pharmaceutical composition according to any one of the above [1] through [5], wherein the disintegrant is at least one selected from the group consisting of crospovidone, low-substituted hydroxypropyl cellulose, croscarmellose sodium, and carmellose calcium,
[7] the controlled-release pharmaceutical composition according to any one of the above [1] through [6], wherein the water-insoluble polymer is at least one selected from the group consisting of ethyl cellulose, an aminoalkyl methacrylate copolymer RS (Eudragit RS), and shellac,
[8] the controlled-release pharmaceutical composition according to any one of the above [1] through [7], wherein the enteric polymer is at least one selected from the group consisting of hydroxypropyl methyl cellulose phthalate, hydroxypropyl methyl cellulose acetate succinate, a methacrylic acid-methyl methacrylate copolymer (Eudragit L, Eudragit S), and a methacrylic acid-ethyl acrylate copolymer (Eudragit LD),
[9] the controlled-release pharmaceutical composition according to any one of the above [1] through [8], wherein the hydrophobic wax is at least one selected from the group consisting of magnesium stearate, calcium stearate, stearic acid, carnauba wax, and a hydrogenated oil,
[10] the controlled-release pharmaceutical composition according to any one of the above [1] through [9], wherein the water-insoluble polymer is ethyl cellulose, the enteric polymer is a methacrylic acid-methyl methacrylate copolymer (Eudragit L, Eudragit S), and the hydrophobic wax is magnesium stearate or calcium stearate,
[11] the controlled-release pharmaceutical composition according to any one of the above [2] through [10], wherein the plasticizer is at least one selected from the group consisting of triethyl citrate, cetyl alcohol, glycerol fatty acid ester, and propylene glycol,
[12] the controlled-release pharmaceutical composition according to any one of the above [1] through [11], wherein a total amount of the water-insoluble polymer and the enteric polymer in the release-controlling coating is 40 to 90 wt%, based on the weight of the release-controlling coating,
[13] the controlled-release pharmaceutical composition according to any one of the above [1] through [12], wherein an amount of the hydrophobic wax in the release-controlling coating is 10 to 60 wt%, based on the weight of the release-controlling coating,
[14] the controlled-release pharmaceutical composition according to any one of the above [1] through [13], wherein an amount of the water-insoluble polymer in the release-controlling coating is 3.0 to 95 wt%, based on the total amount of the water-insoluble polymer and the enteric polymer in the release-controlling coating,
[15] the controlled-release pharmaceutical composition according to any one of the above [2] through [14], wherein an amount of the plasticizer in the release-controlling coating is 0.1 to 20 wt%, based on the weight of the release-controlling coating,
[16] the controlled-release pharmaceutical composition according to any one of the above [1] through [15], wherein the acid-unstable physiologically active substance is a benzimidazole-based compound or a physiologically acceptable salt thereof,
[17] the controlled-release pharmaceutical composition according to the above [16], wherein the benzimidazole-based compound or physiologically acceptable salt thereof is rabeprazole, omeprazole, pantoprazole, lansoprazole or esomeprazole, or a physiologically acceptable salt thereof,
[18] the controlled-release pharmaceutical composition according to the above [16] or [17], wherein the benzimidazole-based compound or physiologically acceptable salt thereof is rabeprazole sodium,
[19] the controlled-release pharmaceutical composition according to any one of the above [3] through [18], wherein the alkaline additive is at least one selected from the group consisting of sodium hydroxide, potassium hydroxide, magnesium oxide, calcium oxide, magnesium hydroxide, and calcium hydroxide,
[20] the controlled-release pharmaceutical composition according to any one of the above [1] through [19], wherein the controlled-release pharmaceutical composition is a tablet, a granular preparation, or a fine granular preparation.
   Moreover, in a second aspect, the present invention provides:
[21] a capsule preparation, comprising: the controlled-release pharmaceutical composition according to any one of the above [1] through [20]; and an enteric pharmaceutical composition in which a core containing an acid-unstable physiologically active substance is covered with an enteric coating,
[22] a pharmaceutical composition package contained in a packaging container, comprising: the controlled-release pharmaceutical composition according to any one of the above [1] through [20]; and an enteric pharmaceutical composition in which a core containing an acid-unstable physiologically active substance is covered with an enteric coating, wherein both of the composition are present in the same packaging container,
[23] a pharmaceutical composition package contained in a packaging container, comprising: the capsule preparation according to the above [21],
[24] the pharmaceutical composition package according to the above [22] or [23], wherein the packaging is sachet or blister packaging.
   Furthermore, in a third aspect, the present invention provides:
[25] a method for producing a controlled-release pharmaceutical composition comprising: forming a release-controlling coating by spraying a solution containing a mixture of a water-insoluble polymer, an enteric polymer and a hydrophobic wax onto a core containing an acid-unstable physiologically active substance and a disintegrant to form a coating covering the core,
[26] the method for producing a controlled-release pharmaceutical composition according to the above [25], wherein the release-controlling coating further comprises a plasticizer,
[27] the method for producing a controlled-release pharmaceutical composition according to the above [25] or [26], wherein the core further comprises an alkaline additive,
[28] the method for producing a controlled-release pharmaceutical composition according to any one of the above [25] through [27], further comprising forming an inert intermediate coating between the core and the release-controlling coating,
[29] the method for producing a controlled-release pharmaceutical composition according to any one of the above [25] through [28], wherein the controlled-release pharmaceutical composition is a pulsed-release pharmaceutical composition.
   Furthermore, in a fourth aspect, the present invention provides:
[30] a method of controlling release to reduce variation in a dissolution lag time, comprising: covering a core containing an acid-unstable physiologically active substance and a disintegrant with a release-controlling coating containing a water-insoluble polymer, an enteric polymer and a hydrophobic wax.

The term "acid-unstable physiologically active substance" used in the present invention means a physiologically active substance having a characteristic of being chemically unstable and thus readily decomposing at an acidic pH in the stomach and/or at an acidic pH. Moreover, the term "inert intermediate coating" used in the present invention means a coating that does not have an adverse effect on the stability of the acid-unstable physiologically active substance contained in the core. Furthermore, the term "lag time" used in the present invention means the time taken for the pharmaceutical composition to start to dissolve out in the solution in vitro, and means the time from taking the pharmaceutical composition to dissolution in vivo.

### Advantageous Effects of the Invention

According to the present invention, in the case of a controlled-release pharmaceutical composition, particularly a pulsed-release pharmaceutical composition, containing an acid-unstable physiologically active substance, a pharmaceutical composition having little variation in dissolution lag time and percentage of dissolution over time, and high reliability of dissolution characteristics can be realized. In particular, with the controlled-release pharmaceutical composition according to the present invention, the dissolution and absorptivity of the active ingredient are good, and moreover the pharmaceutical composition itself has excellent moisture resistance.

### Brief Description of Drawing

FIG. 1 shows a schematic sectional view of a controlled-release pharmaceutical composition according to the present invention;
FIG. 2 shows results of evaluation by dissolution test (1) of rabeprazole sodium in controlled-release pharmaceutical compositions of Examples 1 to 3 according to the present invention;
FIG. 3 shows results of evaluation by dissolution test (1) of rabeprazole sodium in controlled-release pharmaceutical compositions of Examples 4 to 7 according to the present invention;
FIG. 4 shows results of evaluation by dissolution test (1) of rabeprazole sodium in controlled-release pharmaceutical compositions of Examples 8 to 10 according to the present invention;
FIG. 5 shows results of evaluation by dissolution test (1) of rabeprazole sodium in controlled-release pharmaceutical compositions of Controls 1 to 3 used in the present invention;
FIG. 6 shows the relationship between release-controlling coating amount and dissolution lag time in dissolution test (1) of rabeprazole sodium in the controlled-release pharmaceutical compositions of Examples 1 to 6 according to the present invention;
FIG. 7 shows results of dissolution lag times for Examples 11 and 12 according to the present invention as evaluated by dissolution test (2) and dissolution test (1);
FIG. 8 shows results comparing the dissolution lag times between before test commencement and after storing for 2 weeks at 60°C for controlled-release pharmaceutical compositions of Examples 1 to 3 according to the present invention;
FIG. 9 shows results of the dissolution lag times in a dissolution test solution of pH 6.8 and a dissolution test solution of pH 8 for Examples 1 to 3 according to the present invention and Controls 4 to 7;
FIG. 10 shows results of the dissolution lag times in a dissolution test solution of pH 6.8 and a dissolution test solution of pH 8 for Examples 11 and 12 according to the present invention;
FIG. 11 shows results of visual inspection in an external appearance test for Examples 1 to 3 and Examples 13 to 15 according to the present invention;
FIG. 12 shows results comparing dissolution lag times between just after production and after storing for 1 week at 60°C for the controlled-release pharmaceutical compositions of Examples 13 to 15 according to the present invention;
FIG. 13 shows results of changes in concentration in the blood in beagles after administration of the controlled-release pharmaceutical compositions of Examples 11 and 12 according to the present invention;
FIG. 14 shows the correlation between in vitro and in vivo for controlled-release pharmaceutical compositions according to the present invention;
FIG. 15 shows results of the dissolution lag time obtained for Example 16; and
FIG. 16 shows changes in concentration of rabeprazole sodium in the blood in the case of administering the enteric pharmaceutical composition according to Example 16 to a beagle.

### Best Mode for Carrying Out the Invention

The following embodiments are illustrative to explain the present invention, and the present invention is not limited to only these embodiments. The present invention can be carried out in various forms so long as the gist of the present invention is not deviated from.

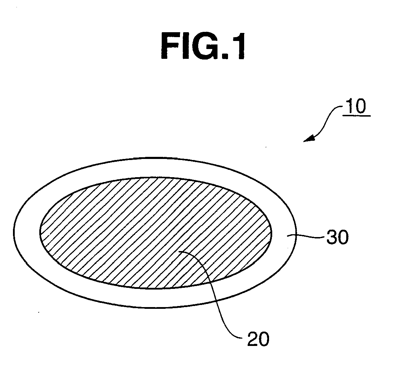

FIG. 1 shows a schematic sectional view of a controlled-release pharmaceutical composition 10 according to the present invention. As shown in FIG. 1, the controlled-release pharmaceutical composition 10 according to the present invention comprises a core 20 containing an acid-unstable physiologically active substance and a disintegrant, and a release-controlling coating 30 which covers the core, and which contains a water-insoluble polymer, an enteric polymer and a hydrophobic wax. Although not shown in FIG. 1, in a preferable form of the present invention, the controlled-release pharmaceutical composition according to the present invention further comprises an inert intermediate coating between the core 20 and the release-controlling coating 30.

There are no particular limitations on the acid-unstable physiologically active substance used in the present invention, but specific examples include a gastric ulcer-treating drug, an antibiotic, an analgesic, an anti-dementia drug, an anti-platelet drug, an antidepressant, a cerebral circulation/metabolism ameliorant, and an antiallergic drug. Examples of publicly known gastric ulcer-treating drug include benzimidazole-based compounds that have a proton pump inhibitory action and strongly suppress gastric acid secretion and physiologically acceptable salts thereof, specifically rabeprazole (I), omeprazole (II), esomeprazole (III), lansoprazole (IV), pantoprazole (V) and tenatoprazole (VI) represented by the chemical formulae shown below and alkali metal salts or alkaline earth metal salts thereof. As an alkali metal salt, a sodium salt or a potassium salt is preferable, and as an alkaline earth metal salt, a magnesium salt is preferable. A particularly preferable gastric ulcer-treating drug is rabeprazole sodium.

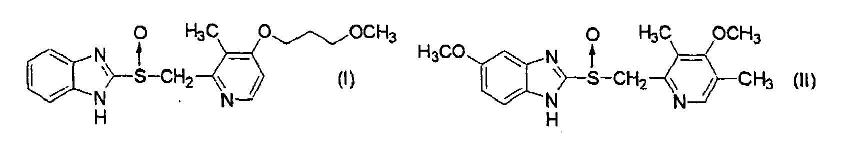

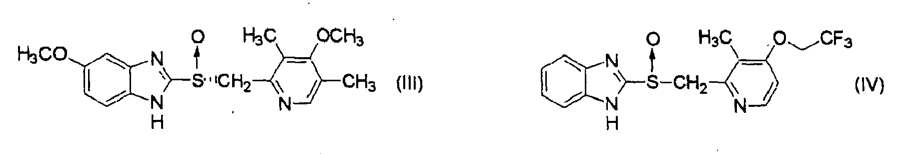

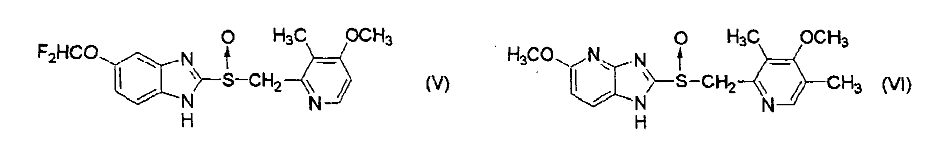

A benzimidazole-based compound used in the present invention can be produced using a publicly known method. For example, the benzimidazole-based compound can be produced using one of the methods disclosed in Japanese Patent Publication Laid-open No. S52-62275, Japanese Patent Publication Laid-open No. S54-141783, Japanese Patent Publication Laid-open No. H1-6270 and so on. More specifically, rabeprazole (I) can be produced according to the method described in the specification of U.S. Patent No. 5045552, omeprazole (II) according to the method described in the specification of U.S. Patent No. 4255431, esomeprazole (III) according to the method described in the specification of U.S. Patent No. 5948789, lansoprazole (IV) according to the method described in the specification of U.S. Patent No. 4628098, pantoprazole (V) according to the method described in the specification of U.S. Patent No. 4758579, and tenatoprazole (VI) according to the method described in the specification of U.S. Patent No. 4808596.

The controlled-release pharmaceutical composition according to the present invention is preferably made to contain at least one alkaline additive in the core as a stabilizer for the acid-unstable physiologically active substance. For example, a benzimidazole-based compound as described above is very unstable in an acidic state, and a pharmaceutical composition containing such a benzimidazole-based compound has a characteristic of readily undergoing discoloration due to production of decomposition products under high-temperature high-humidity conditions. Moreover, benzimidazole-based compounds are unstable in an acidic pH region, but the stability in a neutral pH region varies according to the drug; for example, the half-life at pH 7 is 23 hours for omeprazole, 13 hours for lansoprazole, 39 hours for pantoprazole, and 30 minutes for rabeprazole. Rabeprazole or the like may thus decompose upon intestinal juice penetrating into the core. The stability of the acid-unstable physiologically active substance can thus be secured by adding an alkaline additive such as sodium hydroxide into the core so that the inside of the core will remain alkaline even if intestinal juice penetrates therein. There are no particular limitations on the alkaline additive, but specific examples include sodium hydroxide, potassium hydroxide, magnesium oxide, calcium oxide, magnesium hydroxide, calcium hydroxide, sodium carbonate, sodium phosphate and potassium carbonate, with sodium hydroxide, potassium hydroxide, magnesium oxide, calcium oxide, magnesium hydroxide and calcium hydroxide being preferable, and sodium hydroxide and/or magnesium oxide being particularly preferable.

The amount added of the alkaline additive represented by sodium hydroxide and potassium hydroxide is generally 0.1 to 40 wt%, preferably 1.0 to 20 wt%, more preferably 2.0 to 15 wt%, based on the weight of the benzimidazole-based compound. Furthermore, in the case of using an alkaline additive other than sodium hydroxide or potassium hydroxide, this amount is generally 10 to 5000 wt%, preferably 100 to 2000 wt%, more preferably 200 to 1000 wt%, based on the weight of the benzimidazole-based compound.

The "core" according to the present invention means a core substance that contains the physiologically active substance alone, or also contains at least one pharmaceutical composition additive, and generally has the form of a tablet, granules, fine granules or the like.

There are no particular limitations on the disintegrant contained in the core in the present invention, so long as this disintegrant has a characteristic of expanding the volume upon absorbing water; the core contains at least one such disintegrant. Although there are no particular limitations, specific examples of disintegrants that can be used in the present invention include crospovidone, low-substituted hydroxypropyl cellulose, croscarmellose sodium and/or carmellose calcium, with crospovidone or low-substituted hydroxypropyl cellulose being particularly preferable. In particular, with the benzimidazole-based compound, crospovidone not only has a swelling characteristic as a disintegrant, but also has a marked stabilization effect of suppressing discoloration due to decomposition of the benzimidazole-based compound, and is thus particularly preferable. The amount added of the disintegrant is generally 1 to 50 wt%, preferably 5 to 40 wt%, particularly preferably 10 to 35 wt%, based on the weight of the core. In particular, in the case of using crospovidone with the benzimidazole-based compound, the amount added of the crospovidone is preferably 10 to 1000 wt%, more preferably 20 to 800 wt%, yet more preferably 50 to 500 wt%, most preferably 100 to 300 wt%, based on the weight of the benzimidazole-based compound.

The core may be made to contain any of various other pharmaceutical composition additives, for example, an excipient, a binder, and a lubricant, which are commonly known and so on, can be used as appropriate.

The core in the present invention can be produced using a commonly used method. For example, sodium hydroxide, crospovidone or the like as a stabilizer is mixed with the benzimidazole-based compound, the excipient, the binder and so on are added, and wet granulation such as high shear granulation or extrusion granulation, or dry granulation is carried out. The disintegrant, a lubricant and so on are then added as required, and compression into a tablet is carried out, whereby the core can be produced. There is of course no limitation to such a method.

The coating that covers the core in the present invention is a release-controlling coating containing a water-insoluble polymer, an enteric polymer and a hydrophobic wax. In the present invention, in the case of a controlled-release pharmaceutical composition, particularly a pulsed-release pharmaceutical composition, containing an acid-unstable physiologically active substance, by coating the core with the release-controlling coating containing the water-insoluble polymer, the enteric polymer and the hydrophobic wax, a pharmaceutical composition having little variation in dissolution lag time and high reliability of dissolution characteristics can be produced. That is, the controlled-release pharmaceutical composition having little variation in percentage of dissolution over time and dissolution lag time within a lot or between lots in the same test solution, and having little variation in percentage of dissolution and dissolution lag time with various pH in test solutions is made possible. Furthermore, due to using such a release-controlling coating, the controlled-release pharmaceutical composition according to the present invention is a controlled-release pharmaceutical composition for which changes in external appearance (e.g. cracks in the coating) do not arise even upon being left under high-humidity conditions.

There are no particular limitations on the water-insoluble polymer used in the present invention so long as this water-insoluble polymer has the characteristic of hardly dissolving in water but dissolving or uniformly dispersing in organic solvents such as methanol, ethanol, propanol, isopropanol and acetone. Preferable examples include ethyl cellulose, an aminoalkyl methacrylate copolymer RS (Eudragit RS's (manufactured by Röhm Pharma)) and/or shellac, with ethyl cellulose being particularly preferable. In the present invention, these can be used singly or a plurality can be used in combination.

There are no particular limitations on the enteric polymer used in the present invention, but an example is at least one polymer selected from the group consisting of hydroxypropyl methyl cellulose phthalate, hydroxypropyl methyl cellulose acetate succinate, a methacrylic acid-methyl methacrylate copolymer (Eudragit L (manufactured by Röhm Pharma), Eudragit S (manufactured by Röhm Pharma)) and a methacrylic acid-ethyl acrylate copolymer (Eudragit LD (manufactured by Röhm Pharma)); a methacrylic acid-methyl methacrylate copolymer (Eudragit L, Eudragit S) and/or a methacrylic acid-ethyl acrylate copolymer (Eudragit LD) is preferable, with a methacrylic acid-methyl methacrylate copolymer (Eudragit L) being particularly preferable.

The hydrophobic wax used in the present invention is a hydrophobic additive that has ductility and a lubricant effect; examples include 1) a higher fatty acid having at least 10 carbon atoms and an alkaline earth metal salt thereof and an ester thereof, and 2) wax and so on. There are no particular limitations, but specific examples of 1) and 2) include magnesium stearate, calcium stearate, stearic acid, carnauba wax, glyceryl dibehenate, sucrose fatty acid esters and glycerol fatty acid esters having an HLB value of not more than 5, white beeswax, a hydrogenated oil, and waxes such as microcrystalline wax. The hydrophobic wax is preferably at least one selected from the group consisting of magnesium stearate, calcium stearate, stearic acid, carnauba wax, glyceryl dibehenate and a hydrogenated oil, with magnesium stearate or calcium stearate being particularly preferable.

The dissolution lag time for the release-controlling coating can be controlled through the composition of the release-controlling coating (the proportions of the water-insoluble polymer, the enteric polymer and the hydrophobic wax) and the thickness of the coating. For example, if an amount of the water-insoluble polymer in the release-controlling coating is increased, then the dissolution lag time will become longer, whereas if an amount of the hydrophobic wax is increased, then the dissolution lag time can be made shorter. Moreover, upon increasing the thickness of the coating, the dissolution lag time will become longer.

There are no particular limitations on the amount of the water-insoluble polymer in the release-controlling coating, but this amount is generally 3.0 to 95 wt%, preferably 5.0 to 90 wt%, more preferably 10 to 85 wt%, based on the total amount of the water-insoluble polymer and the enteric polymer in the release-controlling coating. Moreover, there are no particular limitations on the total amount of the water-insoluble polymer and the enteric polymer in the release-controlling coating, but this total amount is generally 30 to 85 wt%, preferably 40 to 75 wt%, more preferably 50 to 65 wt%, based on the weight of the release-controlling coating.

There are no particular limitations on the amount of the hydrophobic wax in the release-controlling coating, but this amount is generally 5 to 65 wt%, preferably 8 to 50 wt%, more preferably 10 to 35 wt%, particularly preferably 20 to 35 wt%, based on the weight of the release-controlling coating.

In a preferable form of the present invention, the release-controlling coating contains ethyl cellulose as the water-insoluble polymer, a methacrylic acid-methyl methacrylate copolymer (Eudragit L, Eudragit S) as the enteric polymer, and magnesium stearate or calcium stearate as the hydrophobic wax.

Furthermore, the release-controlling coating according to the present invention is preferably made to contain a plasticizer. There are no particular limitations on the plasticizer used in the present invention, but specific examples include triethyl citrate, cetyl alcohol, a glycerol fatty acid ester, and propylene glycol; one of these may be used, or a plurality may be used in combination. Cetyl alcohol or triethyl citrate is preferable. In the case that the proportion added of the water-insoluble polymer based on the total amount added of the water-insoluble polymer and the enteric polymer is high, it is preferable to add cetyl alcohol as the plasticizer, whereas in the case that the proportion added of the water-insoluble polymer is low, it is preferable to add triethyl citrate as the plasticizer. There are no particular limitations on the amount of the plasticizer in the release-controlling coating, but this amount is generally 0.1 to 20 wt%, preferably 0.5 to 15 wt%, more preferably 1.0 to 15 wt%, based on the weight of the release-controlling coating. More specifically, in the case that the proportion added of the water-insoluble polymer based on the total amount of the water-insoluble polymer and the enteric polymer is high and hence cetyl alcohol is added, the amount of the cetyl alcohol is generally 0.1 to 10 wt%, preferably 0.5 to 7.0 wt%, more preferably 1.0 to 5.0 wt%, based on the weight of the release-controlling coating. On the other hand, in the case that the proportion added of the water-insoluble polymer based on the total amount of the water-insoluble polymer and the enteric polymer is low and hence triethyl citrate is added, the amount of the triethyl citrate is generally 3.0 to 20 wt%, preferably 6.0 to 15 wt%, more preferably 7.5 to 12 wt%, based on the weight of the release-controlling coating. In particular, in the case that the proportion added of the water-insoluble polymer based on the total amount of the water-insoluble polymer and the enteric polymer is low and hence triethyl citrate is added, it is preferable to add the triethyl citrate in an amount of at least 7.5 wt% based on the weight of the release-controlling coating from the viewpoint of preventing lengthening of the dissolution lag time of the controlled-release pharmaceutical composition according to the present invention.

In the present invention, the covering of the core with the release-controlling coating containing the water-insoluble polymer, the enteric polymer and the hydrophobic wax can be carried out by dissolving or suspending the water-insoluble polymer, the enteric polymer and the hydrophobic wax in a solvent, and using fluidized bed coating, pan coating or the like. Here, the liquid obtained by dissolving or suspending the water-insoluble polymer, the enteric polymer and the hydrophobic wax in the solvent is sprayed into a bed in which the core or a core that has been covered with an inert intermediate coating has been fluidized or agitated, and the solvent is dried off, thus forming the release-controlling coating on the outside of the core or the core that has been covered with the inert intermediate coating.

There are no particular limitations on the solvent of the coating solution containing the water-insoluble polymer, the enteric polymer and the hydrophobic wax used in the present invention, so long as this solvent has the characteristic that the water-insoluble polymer, the enteric polymer and the hydrophobic wax can be dissolved or uniformly dispersed therein. Examples include water, methanol, ethanol, propanol, isopropanol and acetone and the like, with methanol, ethanol, propanol and isopropanol being preferable, and ethanol or isopropanol being particularly preferable. One of these solvents may be used, or a plurality may be used mixed together as appropriate.

The enteric polymer in the release-controlling coating will be acidic, and hence it is undesirable for the enteric polymer to come into direct contact with the benzimidazole-based compound that is the acid-unstable physiologically active substance. In the controlled-release pharmaceutical composition according to the present invention, it is thus preferable to provide an inert intermediate coating that does not have an adverse effect on the stability of the benzimidazole-based compound between the core containing the benzimidazole-based compound and the release-controlling coating containing the water-insoluble polymer, the enteric polymer and the hydrophobic wax. There are no particular limitations on the inert intermediate coating, but this is generally a coating containing a water-soluble polymer, a water-insoluble polymer and/or a water-dispersible substance. There are no particular limitations on the inert intermediate coating used in the present invention, but specific examples include hydroxypropyl cellulose, hydroxypropyl methyl cellulose, an aminoalkyl methacrylate copolymer, ethyl cellulose, lactose, mannitol, and crystalline cellulose and the like. Moreover, the intermediate coating comprising a dispersion of water-insoluble fine particles in a water-insoluble polymer as disclosed in Japanese Patent Publication Laid-open No. H1-29062 may be used.

The controlled-release pharmaceutical composition, particularly a pulsed-release pharmaceutical composition, according to the present invention is a revolutionary pharmaceutical composition having both acid resistance and reliable pulsed dissolution characteristics after a desired dissolution lag time. Regarding the release-controlling coating that contains the water-insoluble polymer, the enteric polymer and the hydrophobic wax and covers the core containing the acid-unstable physiologically active substance and the disintegrant, under acidic conditions the enteric polymer will not dissolve, and hence dissolving out of the physiologically active substance in the core will not occur. Under neutral pH conditions, the enteric polymer will dissolve, and hence small holes will arise in the release-controlling coating, and thus the dissolving liquid will penetrate into the core, and hence the disintegrant contained in the core will swell and cracks will be produced in the release-controlling coating, whereby the physiologically active substance will be dissolved out in a pulsed way. At this time, the hydrophobic wax coexisting with the water-insoluble polymer and the enteric polymer in the release-controlling coating has an action of regulating the strength and fragility of the release-controlling coating, and hence has an action of regulating the dissolution lag time when the physiologically active substance is dissolved out in a pulsed way a desired time after the pharmaceutical composition according to the present invention has been immersed in a solution or internally administered. Accordingly, with the controlled-release pharmaceutical composition, particularly the pulsed-release pharmaceutical composition, according to the present invention, after the set lag time, dissolution occurs with little variation in the dissolution lag time, and there is little variation in the percentage of dissolution over time within a lot or between lots, and hence highly reliable dissolution can be attained.

The dissolution lag time of the controlled-release pharmaceutical composition, particularly the pulsed-release pharmaceutical composition, containing a hydrophobic wax according to the present invention has excellent characteristics, with there being little variation in the characteristics under the same conditions, and the characteristics being little affected by the pH of the dissolving liquid. Moreover, once dissolution starts to take place, the majority of the physiologically active substance dissolves out in a short time. At least 70% of the acid-unstable physiologically active substance generally dissolves out within 3 hours, preferably within 2 hours, more preferably within 1 hour, after the desired dissolution lag time. Consequently, the controlled-release pharmaceutical composition, particularly the pulsed-release pharmaceutical composition, containing a hydrophobic wax according to the present invention has the characteristic of there being very little variation in the dissolution lag time or variation in the percentage of dissolution over time even if the pH in the intestines varies.

From the viewpoint of the dissolution and absorptivity of an active ingredient contained in the pharmaceutical composition and the moisture resistance of the pharmaceutical composition itself, in a preferred aspect of the controlled-release pharmaceutical composition according to the present invention, particularly a pulsed-release pharmaceutical composition, the controlled-release pharmaceutical composition comprises: rabeprazole sodium as the acid-unstable physiologically active substance; the release-controlling coating containing Eudragit L or S and ethyl cellulose with the amount of ethyl cellulose being 10 to 25 wt %, preferably 11 to 20 wt% based on the total amount of Eudragit L or S and ethyl cellulose in the release-controlling coating: calcium stearate with the amount of calcium stearate being 10 to 35 wt%, preferably 20 to 35wt% based on the weight of the release-controlling coating; and triethy citrate with the amount of triethy citrate being 6.0 to 15 wt%, preferably 7.5 to 12 wt% based on the weight of the release-controlling coating.

Example of the form of the controlled-release pharmaceutical composition according to the present invention includes a tablet, a granule, and a fine granule, although there are no particular limitations so long as the pharmaceutical composition is solid.

In the case of a solid pharmaceutical composition for internal administration of the acid-unstable physiologically active substance, the controlled-release pharmaceutical composition according to the present invention may be filled into a capsule together with an enteric pharmaceutical composition in which a core containing the acid-unstable physiologically active substance is covered with an enteric coating, thus producing a capsule preparation. As a result, the patient taking the drug can be given both a fast-acting medical benefit due to the enteric pharmaceutical composition and a sustained medical benefit due to the controlled-release pharmaceutical composition. It is particularly preferable for the controlled-release pharmaceutical composition to be a pulsed-release pharmaceutical composition. That is, a pharmaceutical composition having both a fast-acting effect due to the enteric pharmaceutical composition and ability for the drug to dissolve out after a certain dissolution lag time due to the pulsed-release pharmaceutical composition can be provided. Note that the capsule used in the present invention may be a hard capsule or a soft capsule, and moreover there are no particular limitations on the capsule material, although examples include gelatin, hydroxypropyl methyl cellulose, pullulan and the like. One or a plurality of the controlled-release pharmaceutical composition and one or a plurality of the enteric pharmaceutical composition may be filled into the capsule. For example, a plurality of reduced-diameter mini-tablets of the enteric pharmaceutical composition and a plurality of reduced-diameter mini-tablets of the controlled-release pharmaceutical composition may be filled into a hard capsule, or granules or fine granules of the controlled-release pharmaceutical composition and the enteric pharmaceutical composition may be filled into the capsule, or tablets of the controlled-release pharmaceutical composition and granules or fine granules of the enteric pharmaceutical composition, or granules or fine granules of the controlled-release pharmaceutical composition and tablets of the enteric pharmaceutical composition may be filled into the capsule.

Moreover, the controlled-release pharmaceutical composition according to the present invention may be made into a pharmaceutical composition package in which the controlled-release pharmaceutical composition and an enteric pharmaceutical composition in which a core containing the acid-unstable physiologically active substance is covered with an enteric coating are filled into the same packaging container. There are no particular limitations on the packaging container, although examples are sachet and blister packaging. As a result, the patient taking the drug can be given both a fast-acting medical benefit due to the enteric pharmaceutical composition and a sustained medical benefit due to the controlled-release pharmaceutical composition. It is particularly preferable for the controlled-release pharmaceutical composition to be a pulsed-release pharmaceutical composition. That is, a pharmaceutical composition having both a fast-acting effect due to the enteric pharmaceutical composition and ability for the drug to dissolve out after a certain dissolution lag time due to the pulsed-release pharmaceutical composition can be provided. Moreover, a capsule preparation filled with the controlled-release pharmaceutical composition and an enteric pharmaceutical composition as described above may be filled into a packaging container as described above to produce a pharmaceutical composition package.

Moreover, the present invention also provides a method for producing a controlled-release pharmaceutical composition comprising a step of forming a release-controlling coating by spraying a solution containing a mixture of a water-insoluble polymer, an enteric polymer and a hydrophobic wax onto a core containing an acid-unstable physiologically active substance and a disintegrant to form a coating covering the core. The core may further contain an alkaline additive. Moreover, the release-controlling coating may further contain a plasticizer. Furthermore, to prevent the enteric polymer in the release-controlling coating from coming into direct contact with the acid-unstable physiologically active substance, it is preferable to further include a step of forming an inert intermediate coating between the core and the release-controlling coating. In the present invention, the controlled-release pharmaceutical composition is preferably a pulsed-release pharmaceutical composition.

Furthermore, the present invention also provides a method of controlling release to reduce variation in the dissolution lag time, particularly of a pulsed-release pharmaceutical composition, by covering a core containing an acid-unstable physiologically active substance and a disintegrant with a release-controlling coating containing a water-insoluble polymer, an enteric polymer and a hydrophobic wax.

A controlled-release pharmaceutical composition according to the present invention can, for example, be produced through a method as follows.

### (5 mg tablet of Rabeprazole sodium)

6.72 kg of mannitol, 2.4 kg of crospovidone and 0.5 kg of hydroxypropyl cellulose are added to and mixed with 1.0 kg of rabeprazole sodium, 4 kg of ethanol having 0.1 kg of sodium hydroxide dissolved therein is added, and granulation is carried out. The granules thus produced are dried using a tray dryer, and then passed through a 1.5 mm screen, and then 0.3 kg of crospovidone and 0.18 kg of sodium stearyl fumarate are added and mixed in, and the mixed granules are compressed into tablets using a tablet machine, thus preparing tablets (uncoated tablets) each weighing 56 mg and containing 5 mg of rabeprazole sodium. Next, the uncoated tablets are made to flow in a fluidized bed coating apparatus, and an intermediate coating solution obtained by dissolving 318 g of ethyl cellulose and 540 g of hydroxypropyl cellulose in 16.0 kg of ethanol, and uniformly dispersing 252 g of magnesium stearate into the solution is sprayed on, thus forming an intermediate coating in an amount of 4 mg per tablet, and hence preparing intermediate coating-covered tablets each weighing 60 mg and containing 5 mg of rabeprazole sodium. Moreover, separately, an ethanol solution obtained by dissolving 120 g of Eudragit L100, 480 g of ethyl cellulose and 36 g of cetyl alcohol in 14.26 kg of ethanol, and adding 360 g of magnesium stearate, 90 g of talc and 54 g of titanium dioxide and uniformly dispersing is prepared, and is sprayed onto the intermediate coating-covered tablets flowing in the fluidized bed, thus forming a 10 mg pulsed release-controlling coating, whereby a controlled-release pharmaceutical composition containing 5 mg of rabeprazole sodium in a 70 mg tablet can be produced.

Moreover, when producing such a controlled-release pharmaceutical composition, the uncoated tablets can also be produced using the following composition and production method. For example, 3.0 kg of mannitol, 5.0 kg of magnesium oxide, 0.6 kg of hydroxypropyl cellulose and 0.9 kg of low-substituted hydroxypropyl cellulose are added to and mixed with 1.0 kg of rabeprazole sodium, 3.4 L of ethanol is added, and granulation is carried out. The granules thus produced are dried using a tray dryer, and then passed through a 1.5 mm screen, and then 0.58 kg of low-substituted hydroxypropyl cellulose and 0.12 kg of magnesium stearate are added and mixed in, and the granules are compressed into tablets using a tablet machine, thus preparing tablets (uncoated tablets) each weighing 56 mg and containing 5 mg of rabeprazole sodium.

### (10 mg tablet of Rabeprazole sodium: Production method 1)

5.192 kg of mannitol, 3.96 kg of crospovidone and 0.33 kg of hydroxypropyl cellulose are added to and mixed with 2.2 kg of rabeprazole sodium, 4.4 kg of ethanol having 0.11 kg of sodium hydroxide dissolved therein is added, and granulation is carried out. The granules thus produced are dried using a tray dryer, and then passed through a 1.5 mm screen, and then 0.33 kg of crospovidone and 0.198 kg of sodium stearyl fumarate are added and mixed in, and the granules are compressed into tablets using a tablet machine, thus preparing tablets (uncoated tablets) each weighing 56 mg and containing 10 mg of rabeprazole sodium. Next, the uncoated tablets are made to flow in a fluidized bed coating apparatus, and an intermediate coating solution obtained by dissolving 191 g of ethyl cellulose and 324 g of hydroxypropyl cellulose in 9.58 kg of ethanol and uniformly dispersing 151 g of magnesium stearate into the solution is sprayed on, thus forming an intermediate coating in an amount of 3.7 mg per tablet, and hence preparing intermediate coating-covered tablets each weighing 59.7 mg and containing 10 mg of rabeprazole sodium. Moreover, separately, an ethanol solution is prepared by dissolving 143 g of Eudragit L100, 536 g of ethyl cellulose and 40 g of cetyl alcohol in 13.11 kg of ethanol, and adding 268 g of magnesium stearate, 101 g of talc and 60 g of titanium dioxide and uniformly dispersing, and is sprayed onto the intermediate coating-covered tablets flowing in the fluidized bed, thus forming a 10 mg pulsed release-controlling coating, whereby a controlled-release pharmaceutical composition containing 10 mg of rabeprazole sodium in a 69.7 mg tablet can be produced.

### (Rabeprazole sodium 10 mg tablets: Production method 2)

4.92 kg of mannitol and 3 kg of crospovidone are added to and mixed with 2 kg of rabeprazole sodium, 4 kg of ethanol having 0.1 kg of sodium hydroxide dissolved therein is added, and granulation is carried out. The granules thus produced are dried using a tray dryer, and then passed through a 1 mm screen, and then 0.3 kg of crospovidone and 0.18 kg of sodium stearyl fumarate are added and mixed in, and the granules are compressed into tablets using a tablet machine, thus preparing tablets (uncoated tablets) each weighing 52.5 mg and containing 10 mg of rabeprazole sodium. Next, the uncoated tablets are made to flow in a fluidized bed coating apparatus, and an intermediate coating solution obtained by dissolving 651 g of hydroxypropyl cellulose in 12.52 kg of ethanol and uniformly dispersing 219 g of calcium stearate into the solution is sprayed on, thus forming an intermediate coating in an amount of 2.9 mg per tablet, and hence preparing intermediate coating-covered tablets each weighing 55.4 mg and containing 10 mg of rabeprazole sodium. Moreover, separately, an ethanol solution obtained by dissolving 2.2 kg of Eudragit L100, 275 g of ethyl cellulose and 446 g of triethyl citrate in 55 kg of ethanol, and adding 1485 g of calcium stearate, 372 g of talc and 223 g of titanium dioxide and uniformly dispersing is prepared, and is sprayed onto the intermediate coating-covered tablets flowing in the fluidized bed, thus forming an 8 mg pulsed release-controlling coating, whereby a controlled-release pharmaceutical composition containing 10 mg of rabeprazole sodium in a 63.4 mg tablet can be produced.

### Advantageous Effects of the Invention

According to the present invention, in the case of a controlled-release pharmaceutical composition, particularly a pulsed-release pharmaceutical composition, containing an acid-unstable physiologically active substance, a pharmaceutical composition having little variation in dissolution lag time and percentage of dissolution over time, and high reliability of dissolution characteristics can be prepared. In particular, with the controlled-release pharmaceutical composition according to the present invention, the dissolution and absorptivity of the active ingredient are good, and moreover the pharmaceutical composition itself has excellent moisture resistance. The advantageous effects of the present invention will now be described together with the following experimental examples.

### Experimental Example 1

Effect of reducing variation in percentage of dissolution over time and variation in dissolution lag time for pharmaceutical composition and thus increasing dissolution precision through adding hydrophobic wax to release-controlling coating

Using rabeprazole sodium as the acid-unstable physiologically active substance, controlled-release pharmaceutical compositions having release-controlling coatings of various compositions and coating amounts were prepared following Examples 1 to 8 described below, and dissolution tests were carried out thereon. The composition of the release-controlling coating was adjusted by changing the amounts added of the water-insoluble polymer, the enteric polymer and the hydrophobic wax, and the coating amount was adjusted through the amount coated on.

For the controlled-release pharmaceutical compositions of Examples 1 to 3, the proportions of Eudragit L100 (enteric polymer), ethyl cellulose (water-insoluble polymer), magnesium stearate (hydrophobic wax) and cetyl alcohol in the release-controlling coating were 10.5 wt%, 42.1 wt%, 31.6 wt% (amount of hydrophobic wax in the release-controlling coating based on the weight of release-controlling coating = 31.6 wt%, amount of water-insoluble polymer in release-controlling coating based on the total amount of water-insoluble polymer and enteric polymer in release-controlling coating = 80 wt%) and 3.2 wt% respectively, and the coating amount per tablet (containing 5 mg of rabeprazole sodium) was changed between 10 mg, 15 mg and 20 mg. Moreover, for the controlled-release pharmaceutical compositions of Examples 4 to 6, the proportions of Eudragit L100 (enteric polymer), ethyl cellulose (water-insoluble polymer) and magnesium stearate (hydrophobic wax) in the release-controlling coating were 15 wt%, 40 wt% and 30 wt% respectively (amount of hydrophobic wax in release-controlling coating based on the weight of release-controlling coating = 30 wt%, amount of water-insoluble polymer in release-controlling coating based on the total amount of water-insoluble polymer and enteric polymer in release-controlling coating = 72.7 wt%), and the coating amount per tablet (containing 5 mg of rabeprazole sodium) was changed between 10 mg, 15 mg and 20 mg.

For the controlled-release pharmaceutical composition of Example 7, the proportions of Eudragit L100 (enteric polymer), ethyl cellulose (water-insoluble polymer) and magnesium stearate (hydrophobic wax) in the release-controlling coating were 11.8 wt%, 47.1 wt% and 23.5 wt% respectively (amount of hydrophobic wax in release-controlling coating based on the weight of release-controlling coating = 23.5 wt%, amount of water-insoluble polymer in release-controlling coating based on the total amount of water-insoluble polymer and enteric polymer in release-controlling coating = 80 wt%), and the coating amount per tablet (containing 10 mg of rabeprazole sodium) was made to be 8 mg.

For the controlled-release pharmaceutical composition of Example 11, the proportions of Eudragit L100 (enteric polymer), ethyl cellulose (water-insoluble polymer), calcium stearate (hydrophobic wax) and triethyl citrate in the release-controlling coating were 39.6 wt%, 9.9 wt%, 29.7 wt% (amount of hydrophobic wax in release-controlling coating based on the weight of release-controlling coating = 29.7 wt%, amount of water-insoluble polymer in release-controlling coating based on the total amount of water-insoluble polymer and enteric polymer in release-controlling coating = 20 wt%) and 9 wt% respectively, and the coating amount per tablet (containing 10 mg of rabeprazole sodium) was made to be 8 mg.

For the controlled-release pharmaceutical composition of Example 12, the proportions of Eudragit L100 (enteric polymer), ethyl cellulose (water-insoluble polymer), calcium stearate (hydrophobic wax) and triethyl citrate in the release-controlling coating were 44.0 wt%, 5.5 wt%, 29.7 wt% (amount of hydrophobic wax in release-controlling coating based on the weight of release-controlling coating = 29.7 wt%, amount of water-insoluble polymer in release-controlling coating based on the total amount of water-insoluble polymer and enteric polymer in release-controlling coating = 11.1 wt%) and 8.9 wt% respectively, and the coating amount per tablet (containing 10 mg of rabeprazole sodium) was made to be 8 mg.

For the controlled-release pharmaceutical compositions of Examples 13 to 15, the proportions of Eudragit L100 (enteric polymer), ethyl cellulose (water-insoluble polymer), calcium stearate (hydrophobic wax) and a plasticizer in the release-controlling coating were 42.5 wt%, 7 wt%, 29.7 wt% (amount of hydrophobic wax in release-controlling coating based on the weight of release-controlling coating = 29.7 wt%, amount of water-insoluble polymer in release-controlling coating based on the total amount of water-insoluble polymer and enteric polymer in release-controlling coating = 14.1 wt%) and 8.9 wt% respectively (Example 13: triethyl citrate, Example 14: cetyl alcohol, Example 15: glycerol fatty acid ester), and the coating amount per tablet (containing 10 mg of rabeprazole sodium) was changed between various values (6, 10 and 14 mg).

Moreover, as control experiments regarding pharmaceutical compositions covered with a coating not containing a hydrophobic wax (i.e. containing a water-insoluble polymer and an enteric polymer), pharmaceutical compositions having coatings of various compositions and coating amounts were prepared following Controls 1 to 3 described below, and evaluation was similarly carried out.

For the pharmaceutical compositions of Controls 1 and 2, the proportions of Eudragit L100 (enteric polymer) and ethyl cellulose (water-insoluble polymer) in the coating were 40 wt% and 40 wt% respectively (hydrophobic wax not contained in release-controlling coating, amount of water-insoluble polymer in release-controlling coating based on the total amount of water-insoluble polymer and enteric polymer in release-controlling coating = 50 wt%), and the coating amount per tablet was made to be 5 or 10 mg. Moreover, for the pharmaceutical composition of Control 3, the proportions of Eudragit L100 (enteric polymer) and ethyl cellulose (water-insoluble polymer) in the coating were 15.4 wt% and 61.5 wt% respectively (hydrophobic wax not contained in release-controlling coating, amount of water-insoluble polymer in release-controlling coating based on the total amount of water-insoluble polymer and enteric polymer in release-controlling coating = 80 wt%), and the coating amount per tablet was made to be 5 mg.

### (Dissolution test (1))

This dissolution test was carried out using the following method for Examples 1 to 7 with n (number of cases) = 2.

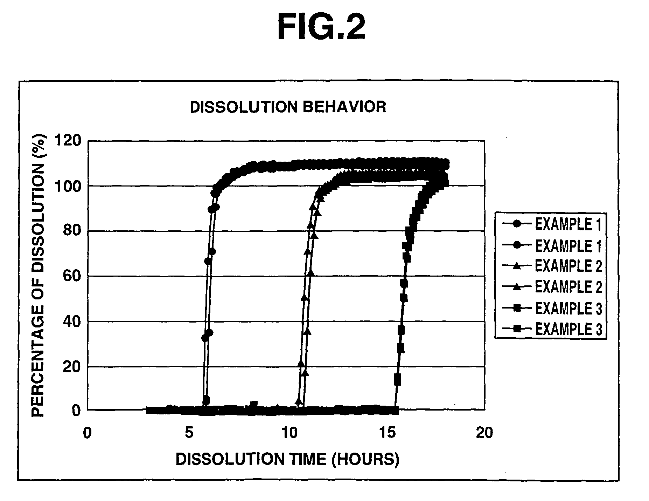

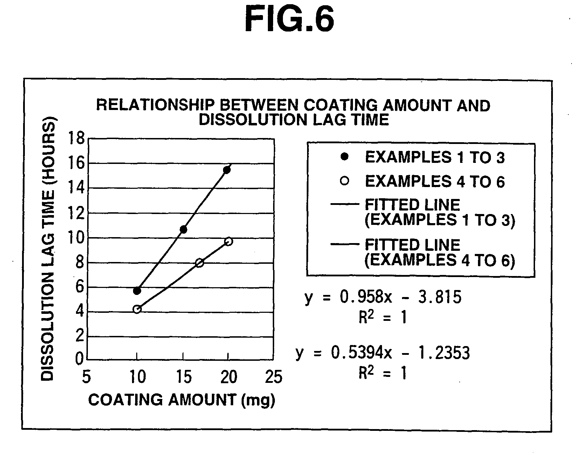

One tablet of the controlled-release pharmaceutical composition was put into 750 mL of a 0.1 N hydrochloric acid solution, and stirring was carried out for 2 hours using a paddle method (50 rpm). After that, 250 mL of a 0.2 M trisodium phosphate solution was immediately added, thus adjusting the pH of the solution to 6.8, and the dissolution test was carried out continuously. Sampling was carried out using a flow cell, and absorbance measurements (wavelength 290 nm) were carried out using an ultraviolet spectrophotometer, thus measuring the change in the percentage of rabeprazole sodium dissolved out over time. The results of the dissolution test are shown in FIGS. 2 to 4, and the correlation between the release-controlling coating amount and the dissolution lag time in FIG. 6. The dissolution lag time indicates the time taken for the drug to start to dissolve out in the test solution at pH 6.8.

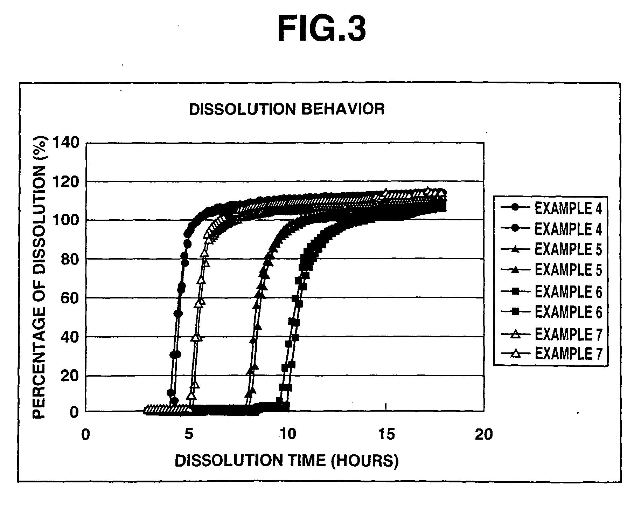

From the results shown in FIGS. 2 and 3, it can be seen that in a dissolution test using test solutions of the same pH under the same conditions, for all of the controlled-release pharmaceutical compositions covered with a release-controlling coating containing a hydrophobic wax, in a dissolution test with n (number of cases) = 2, there was hardly any variation in the pulsed dissolution lag time, and there was little variation in the percentage of dissolution over time, and hence the reproducibility was excellent. Moreover, for the controlled-release pharmaceutical compositions of Examples 1 to 3, compared with the controlled-release pharmaceutical compositions of Examples 4 to 6, the amount of the water-insoluble polymer was high and the amount of the enteric polymer low, based on the total amount of the water-insoluble polymer and the enteric polymer in the release-controlling coating, and hence at the same coating amount, the dissolution lag time was longer for the controlled-release pharmaceutical compositions of Examples 1 to 3 than the controlled-release pharmaceutical compositions of Examples 4 to 6.

For the controlled-release pharmaceutical composition of Example 7, compared with Example 1, the amount added of the hydrophobic wax was lower (Example 7: 23.5%, Example 1: 31.6%), and hence the dissolution lag time tended to be longer, but the pulsed dissolution ability was good.

From the results shown in FIG. 6, it can be seen that a good proportional relationship between the coating amount of the release-controlling coating containing the hydrophobic wax and the dissolution lag time was observed. It is thus possible to produce a controlled-release pharmaceutical composition having a desired dissolution lag time with high precision.

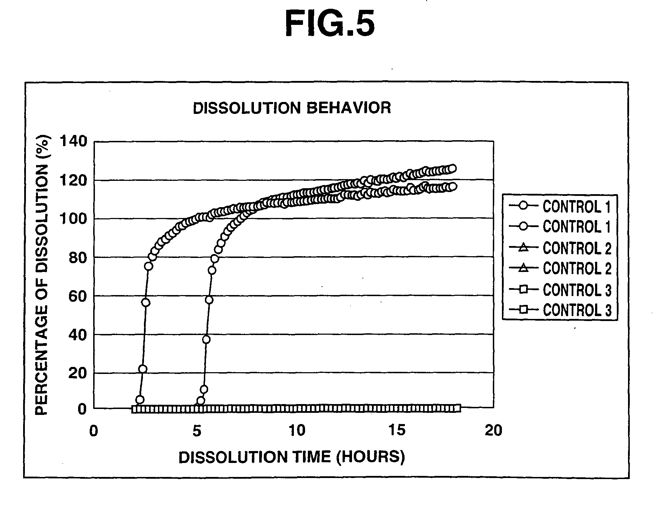

On the other hand, from the results shown in FIG. 5, it can be seen that for Controls 1 to 3 covered with a coating not containing a hydrophobic wax, there was great variation in the dissolution lag time, and moreover the dissolution lag time changed greatly upon slight differences in the composition. Specifically, for the pharmaceutical composition of Control 1 (coating amount 5 mg), as shown in FIG. 5, pulsed dissolution was observed, but there was great variation in the dissolution lag time with n = 2 (the respective dissolution lag times were 2.5 hours and 5.5 hours). Moreover, for the pharmaceutical composition of Control 2 (coating amount 10 mg), dissolving out of the rabeprazole sodium was not observed at all up to 15 hours from the start of the dissolution test. Furthermore, for the pharmaceutical composition of Control 3 (coating amount 5 mg) in which the amount of the water-insoluble polymer based on the total amount of the water-insoluble polymer and the enteric polymer in the release-controlling coating was the same as in Examples 1 to 3 (80%), again dissolving out of the rabeprazole sodium was not observed at all up to 15 hours from the start of the dissolution test. For the pharmaceutical compositions covered with a coating not containing a hydrophobic wax, under physiological conditions (pH not more than 7.4), there was great variation in the dissolution lag time, and moreover the dissolution lag time changed greatly upon slight differences in the composition.

### (Dissolution test (2))

This dissolution test was carried out using the following method for Examples 11 and 12 with n (number of cases) = 6.

One tablet of the controlled-release pharmaceutical composition was put into 750 mL of a 0.1 N hydrochloric acid solution, and stirring was carried out for 2 hours using a paddle method (50 rpm). After that, replacement with a dissolution test solution A (900 mL) that had been kept at 37°C in advance was carried out immediately, and the dissolution test was carried out continuously. Sampling was carried out using a flow cell, and absorbance measurements (wavelength 290 nm) were carried out using an ultraviolet spectrophotometer, thus measuring the change in the percentage of rabeprazole sodium dissolution over time. Dissolution test solution A: A solution (pH 6.8) obtained by mixing together a 0.1 N hydrochloric acid solution and a 0.2 M trisodium phosphate solution, adjusting the pH to 6.5, and diluting by a factor of 8 with purified water was used.

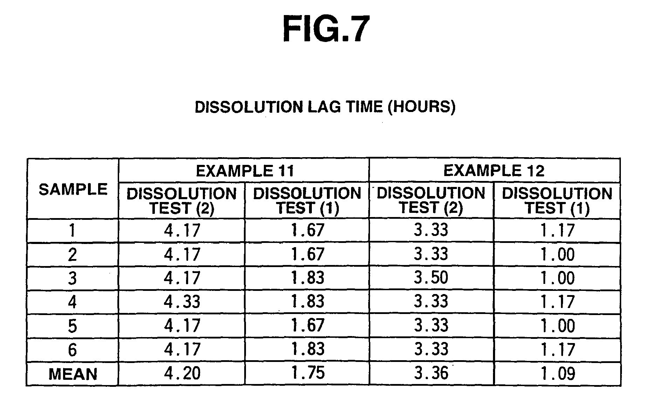

FIG. 7 shows the results of the dissolution lag time for Examples 11 and 12 according to the present invention as evaluated by dissolution test (2), and the results of the dissolution lag time for the same samples as evaluated by dissolution test (1). In FIG. 7, the results are shown separately for each sample. From the results shown in FIG. 7, it can be seen that there is little variation in the dissolution lag time between the samples for both Example 11 and Example 12.

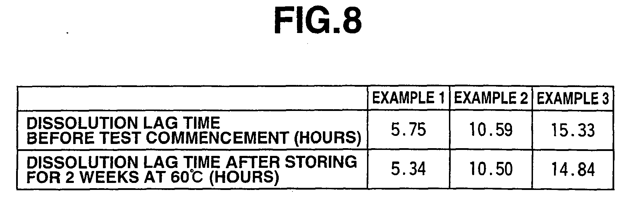

Next, for the pharmaceutical compositions of Examples 1 to 3, 30 tablets of each and 1 g of a desiccant were put into a polyethylene bottle, the cap was put on, and the tablets were stored for two weeks at 60°C. A dissolution test (test method (1)) was then carried out on the samples, and the dissolution lag time was determined. From the results shown in FIG. 8, it can be seen that there was hardly any change in the dissolution lag time (mean value with n = 2). Moreover, no change in the external appearance of the pharmaceutical compositions of Examples 1 to 3 was observed upon leaving for one week under high-humidity conditions.

It was clear that due to adding a hydrophobic wax into the release-controlling coating, a controlled-release pharmaceutical composition of an acid-unstable physiologically active substance according to the present invention has reliable dissolution characteristics, with there being little variation in dissolution lag time, and little variation in percentage of dissolution over time, and hence the dissolution precision in terms of reproducibility and so on being excellent. According to the present invention, the controlled-release pharmaceutical composition having a desired dissolution lag time to high precision can be produced.

### Experimental Example 2

Effect of reducing variation in dissolution lag time with pH of dissolving liquid and thus increasing dissolution precision through adding hydrophobic wax to release-controlling coating

Using rabeprazole sodium as the acid-unstable physiologically active substance, dissolution tests were carried out at pH 6.8 and pH 8 for Examples 1 to 3 described below. Moreover, as control experiments, regarding pharmaceutical compositions covered with a coating not containing a hydrophobic wax (i.e. containing a water-insoluble polymer and an enteric polymer), pharmaceutical compositions having coatings of various compositions and coating amounts were prepared following Controls 4 to 7 described below, and evaluation was similarly carried out.

For the pharmaceutical compositions of Controls 4 to 7, the proportions of Eudragit L100 (enteric polymer) and ethyl cellulose (water-insoluble polymer) in the coating were 42.1 wt% and 42.1 wt% respectively (hydrophobic wax not contained in release-controlling coating, amount of water-insoluble polymer in release-controlling coating based on the total amount of water-insoluble polymer and enteric polymer in release-controlling coating = 50 wt%), and the coating amount per tablet was changed between 15 mg, 20 mg, 25 mg and 30 mg.

The dissolution test at pH 6.8 was carried out using the method of dissolution test (1) described earlier. Moreover, the dissolution test at pH 8 was carried out using the method described below (dissolution test (3)). Note that for Examples 1 to 3 and Controls 4 to 7, n (number of cases) = 2, and for Examples 11 and 12, n (number of cases) = 6.

### (Dissolution test (3) at pH 8)

One tablet of the controlled-release pharmaceutical composition was put into 700 mL of a 0.1 N hydrochloric acid solution, and stirring was carried out for 2 hours using a paddle method (50 rpm). After that, 300 mL of a 0.57 mol/L 2-amino-2-hydroxymethyl-1,3-propanediol solution was immediately added, thus adjusting the pH of the solution to 8, and the dissolution test was carried out continuously. For the sampling liquid, the percentage of rabeprazole sodium dissolved out was measured over time using HPLC.
HPLC conditions
Mobile phase: Methanol / 50 mmol/L phosphate buffer (pH 7.0) mixed liquid (60:40, V/V)
Column: ODS column (YMC, 4.6 mm diameter x 150 mm)
Detection: 290 nm

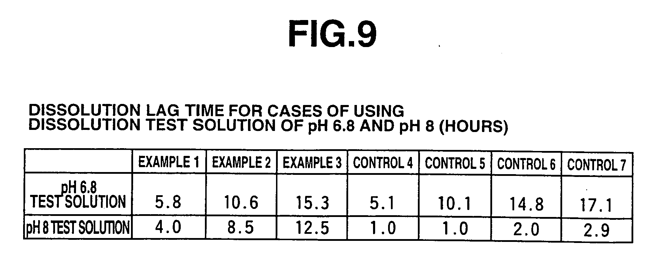

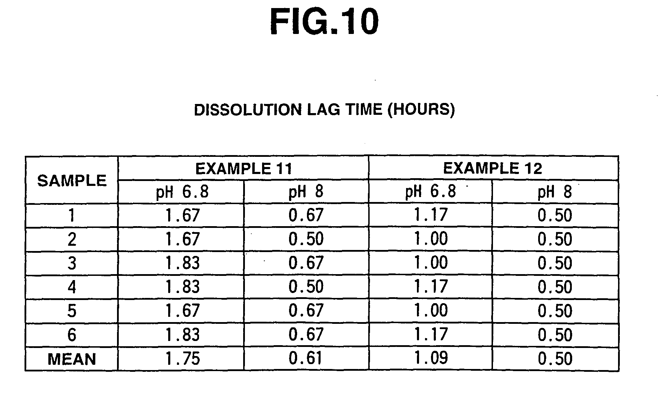

FIGS. 9 and 10 show the dissolution lag times for the case of using the dissolution test solution of pH 6.8 and the dissolution lag time for the case of using the dissolution test solution of pH 8 (mean with n = 2), respectively. Note that the dissolution tests with the two different pH's were carried out in consideration of the ionic strength of the digestive juice in the gastrointestinal tract in vivo and so on.

From the results shown in FIG. 9, it can be seen that for the pulsed-release pharmaceutical compositions of Examples 1 to 3, no significant difference was observed in the dissolution lag time between the dissolution test solution of pH 6.8 and the dissolution test solution of pH 8. On the other hand, for the pharmaceutical compositions of Controls 4 to 7, the dissolution lag time differed greatly between the dissolution test solution of pH 6.8 and the dissolution test solution of pH 8, with a slight variation in the pH causing a significant variation in the dissolution lag time.

As is clear from the results shown in FIG. 10, for the pulsed-release pharmaceutical compositions of Examples 11 and 12, no significant difference was observed in the dissolution lag time upon changing the pH of the dissolution test solution. Furthermore, comparing Examples 11 and 12, the values obtained for the dissolution lag time are lower for Example 12, and hence it can be seen that if the proportion of the enteric polymer (Eudragit L100) is high, based on the total amount of the enteric polymer and the water-insoluble polymer in the release-controlling coating in the present invention, then the dissolution lag time is shortened.

### Experimental Example 3

### Effect of plasticizer on change in external appearance, and effect of plasticizer on lengthening of dissolution lag time after storage

### Test of change in external appearance

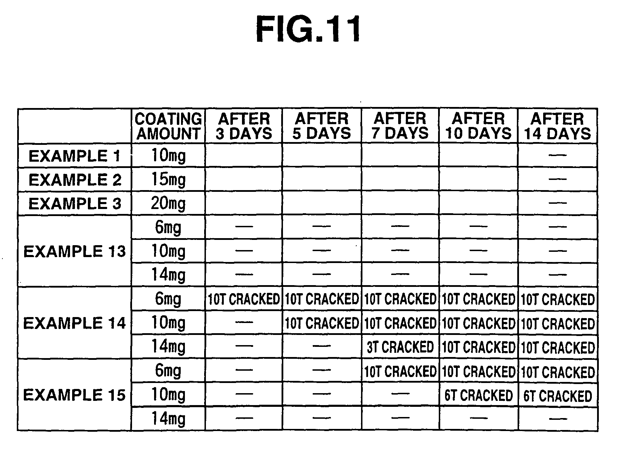

Ten tablets were stored in a desiccator at 75% RH (relative humidity) prepared using a sodium chloride saturated salt solution, and changes in the external appearance over time at 25°C were observed visually. FIG. 11 shows the results of the visual inspection for the various Examples. Note that 'T' in '10T cracked' in FIG. 11 is an abbreviation for "tablet"; "10T cracked" means that cracks were observed in all ten of the tablets, and "3T cracked" means that cracks were observed in three out of the ten tablets.

From the results shown in FIG. 11, it can be seen that for Examples 1 to 3, cracks were not observed upon storing for 2 weeks under conditions of 25°C and 75% RH. In contrast with this, for the pharmaceutical compositions of Examples 13 to 15, although changes in the external appearance are not observed under low-humidity conditions, upon storing under conditions of 25°C and 75% RH, for Examples 14 and 15 there were pharmaceutical compositions for which cracks arose in the surface of the pharmaceutical composition. However, for the pharmaceutical compositions of Example 13, cracks were not observed upon storing for 2 weeks under conditions of 25°C and 75% RH.

### Effect of plasticizer on lengthening of dissolution lag time

Using the controlled-release pharmaceutical compositions of Examples 13 to 15 according to the present invention, a comparison was carried out between the dissolution lag time after storing for 1 week at 60°C and the initial dissolution lag time after production of the controlled-release pharmaceutical composition. For the dissolution tests, the test solution of dissolution test (2) was used.

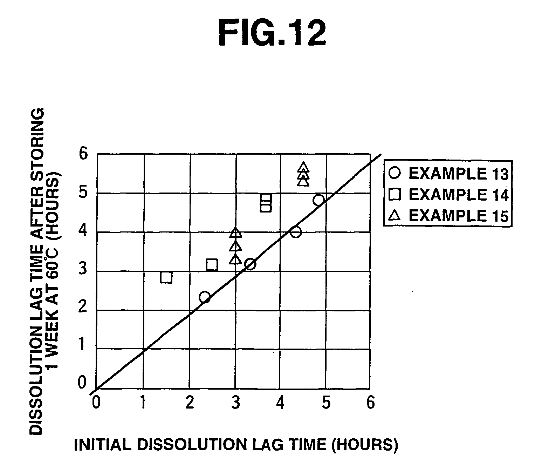

FIG. 12 shows results comparing the dissolution lag time between just after production and after storing for 1 week at 60°C for the controlled-release pharmaceutical compositions of Examples 13 to 15 according to the present invention. Just after production, measurement was carried out with n (number of cases) = 2, and the mean value was taken as the value on the horizontal axis. As is clear from the results of FIG. 12, for Example 13 of the present invention, lengthening of the dissolution lag time after storage under severe conditions was not observed. It can thus be seen that for a release-controlling coating containing a water-insoluble polymer and an enteric polymer, in the case that the proportion of the water-insoluble polymer based on the total amount of the water-insoluble polymer and the enteric polymer is low, if triethyl citrate is added as a plasticizer, then lengthening of the dissolution lag time for the controlled-release pharmaceutical composition according to the present invention is prevented.

From the above description, it was clear that the controlled-release pharmaceutical composition of the acid-unstable physiologically active substance according to the present invention has reliable dissolution characteristics, with there being little variation in the dissolution lag time with pH of the dissolving liquid, and the dissolution precision in terms of reproducibility and so on being high.

### Experimental Example 4

### Relationship between in vitro and in vivo for controlled-release pharmaceutical compositions according to the present invention

Using Examples 11 and 12 as shown in FIG. 10, the concentration in the blood was measured in beagles. As the test method, six beagles were used for each of the Examples, the beagles were made to go without food for 12 hours before administration, and pentagastrin was administered 30 minutes before administration. For each beagle, using a capsule filled with six tablets of the pharmaceutical composition of the Example, the controlled-release pharmaceutical composition corresponding to 60 mg was administered. Blood samples were taken from the beagle 1 to 13 hours and 24 hours after administration of the pharmaceutical composition, and the rabeprazole sodium concentration was measured using HPLC with conditions as in dissolution test (3) described earlier.

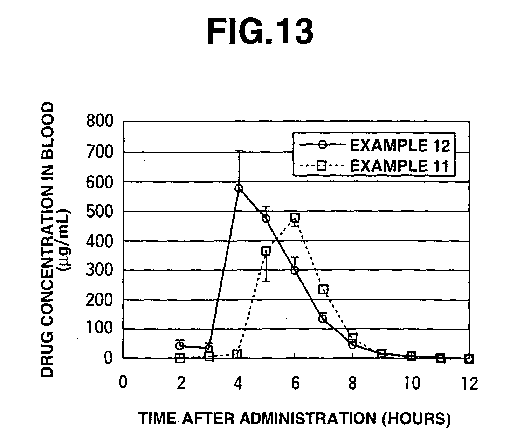

FIG. 13 shows the results of the changes in the concentration in the blood in the beagles after administration of the controlled-release pharmaceutical compositions of Examples 11 and 12 according to the present invention. From the results shown in FIG. 13, it was found that in beagles, compared with Example 11, the dissolution lag time is shorter for the controlled-release pharmaceutical composition of Example 12, and hence the drug is dissolved out more quickly. The results shown in FIG. 13 agree with the in vitro results shown in FIG. 10, thus establishing that the dissolution lag time is indeed shorter if the proportion of the enteric polymer based on the total amount of the enteric polymer and the water-insoluble polymer in the release-controlling coating is higher. Moreover, as is clear from the results shown in FIG. 13, it can be seen that even in beagles, a controlled-release pharmaceutical composition according to the present invention is a pulsed-release pharmaceutical composition.

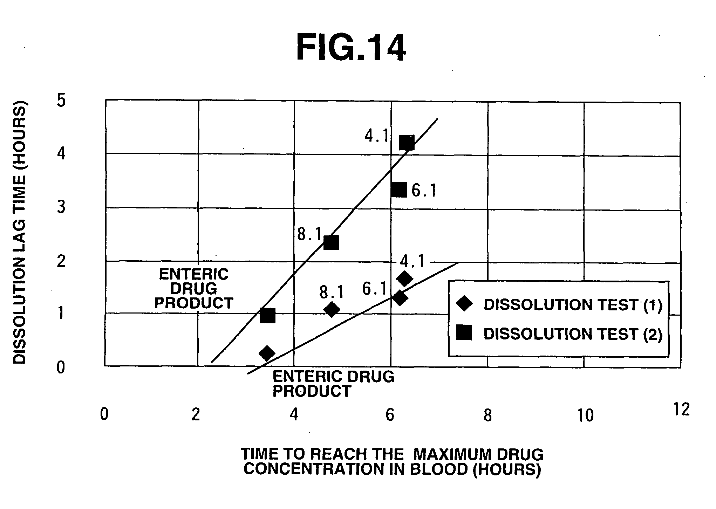

FIG. 14 shows the correlation between in vitro and in vivo for controlled-release pharmaceutical compositions according to the present invention. Regarding the pharmaceutical compositions shown in FIG. 14, the total amount of the enteric polymer (Eudragit L100) and the water-insoluble polymer (ethyl cellulose) in the release-controlling coating was held constant, and '8:1' in FIG. 14 means that the Eudragit L100 : ethyl cellulose proportion was 8:1 (Example 12), '6:1' means that the Eudragit L100 : ethyl cellulose proportion was 6:1, and '4:1' means that the Eudragit L100 : ethyl cellulose proportion was 4:1 (Example 11). Moreover, each of the release-controlling coatings contained 9 wt% of triethyl citrate based on the weight of the release-controlling coating. Furthermore, the 'enteric pharmaceutical composition' was the pharmaceutical composition produced in Example 16 described later.

The values on the horizontal axis shown in FIG. 14 show the mean of the time at which the concentration in the blood reached a maximum when the change over time in the concentration in the blood was evaluated for six beagles using the controlled-release pharmaceutical composition according to the present invention. On the other hand, the values on the vertical axis in FIG. 14 show the dissolution lag times for dissolution test (1) and dissolution test (2). From the results shown in FIG. 14, it is clear that there is a good correlation between the time taken for the drug to start to dissolve out in vitro and the time taken for the concentration of the drug in the blood to reach a maximum in vivo for rabeprazole sodium pharmaceutical compositions having a release-controlling coating containing Eudragit L100, ethyl cellulose and triethyl citrate with a prescribed quantitative relationship therebetween. This suggests that by adjusting the dissolution lag time in vitro, the time at which the concentration in the blood reaches a maximum in vivo can be controlled.

### Experimental Example 5

### Capsule preparation containing enteric pharmaceutical composition and the controlled-release pharmaceutical composition according to the present invention

The enteric pharmaceutical composition of Example 16 is a pharmaceutical composition in which uncoated tablets the same as those described in Example 11 are used, an intermediate coating comprising ethyl cellulose, hydroxypropyl cellulose and magnesium stearate is provided on the uncoated tablets, and then an enteric coating consisting mainly of hydroxypropyl methyl cellulose phthalate is coated on.

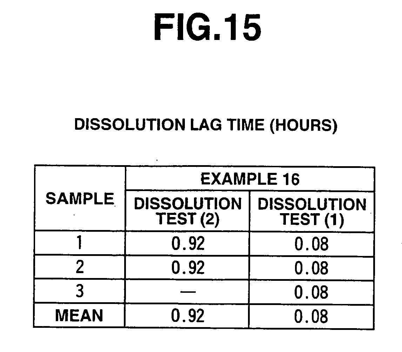

The enteric pharmaceutical composition of Example 16 was evaluated using the methods of dissolution test (2) and dissolution test (1) described earlier. FIG. 15 shows the results of the dissolution lag time obtained for Example 16.

It can be seen that the value of the dissolution lag time for a controlled-release pharmaceutical composition according to the present invention according to the method of dissolution test (2) is significantly higher than the value obtained for Example 16. Accordingly, if the enteric pharmaceutical composition of Example 16 and the controlled-release pharmaceutical composition according to the present invention are filled into a single capsule, and the resulting capsule preparation is administered to a human or an animal such as a beagle, then there can be designed a pharmaceutical composition for which the drug dissolves out from the enteric pharmaceutical composition immediately after administration, and then the drug dissolves out from the controlled-release pharmaceutical composition according to the present invention thereafter.

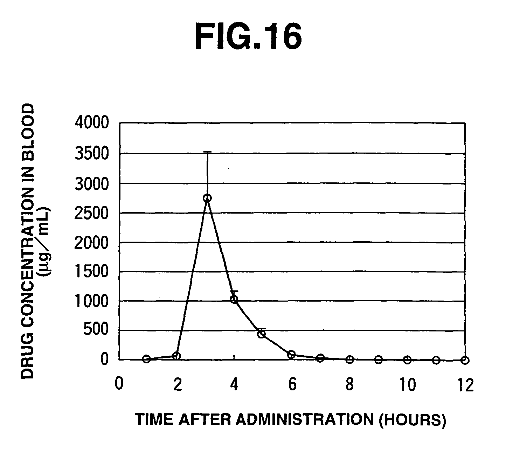

FIG. 16 is a graph showing the changes in the concentration of rabeprazole sodium in the blood in the case of administering the enteric pharmaceutical composition according to Example 16 to a beagle. Note that the results shown in FIG. 16 are results evaluated using the same method as for FIGs. 13 and 14 described earlier. From the results shown in FIG. 16, it can be seen that for the enteric pharmaceutical composition according to Example 16, the concentration of rabeprazole sodium in the blood reaches a maximum approximately 3 hours after administration to a beagle, and has become approximately zero 6 hours after administration.

On the other hand, as shown in FIG. 13, it can be seen that for the controlled-release pharmaceutical composition of Example 11 according to the present invention, the concentration of rabeprazole sodium in the blood reaches a maximum approximately 6 hours after administration to a beagle. From these facts, it is anticipated that if a capsule preparation obtained by filling the enteric pharmaceutical composition of Example 16 and the controlled-release pharmaceutical composition according to the present invention of Example 11 into a single capsule (e.g. an HPMC capsule having hydroxypropyl methyl cellulose as a base material thereof (made by Shionogi Qualicaps)) is administered to a beagle, then rabeprazole sodium will be present in the blood from approximately 2 hours to approximately 9 hours after administration, and hence there can be designed a pharmaceutical composition with a longer medical benefit duration than in the case of administering the enteric pharmaceutical composition or the controlled-release pharmaceutical composition alone. Moreover, by controlling the thickness of the enteric coating of the enteric pharmaceutical composition, or filling controlled-release pharmaceutical compositions according to the present invention having different dissolution lag times into the capsule preparation as appropriate, it becomes possible to select the time of commencement of the medical benefit after taking the capsule preparation and the duration of the medical benefit.

Following is a detailed description of the preparation and so on of examples and controls; however, the present invention is not limited by these examples.

### Example 1

Uncoated tablets of the following composition were produced, an intermediate coating was coated on, and then a release-controlling coating was coated on.

6.72 kg of mannitol, 2.4 kg of crospovidone and 0.5 kg of hydroxypropyl cellulose were added to and mixed with 1.0 kg of rabeprazole sodium, 4 kg of ethanol having 0.1 kg of sodium hydroxide dissolved therein was added, and granulation was carried out. The granules thus produced were dried for 20 hours at 50°C, and then passed through a 1.5 mm screen, and then 0.3 kg of crospovidone and 0.18 kg of sodium stearyl fumarate were added and mixed in, and tablet formation was carried out using a rotary tablet machine, thus obtaining tablets (uncoated tablets) each weighing 56 mg. Next, 3 kg of the tablets were put into a coating pan, and an intermediate coating solution of the following composition was sprayed on, thus forming an intermediate coating in an amount of 3.7 mg per tablet. The intermediate coating solution was prepared by dissolving 318 g of ethyl cellulose and 540 g of hydroxypropyl cellulose in 16.0 kg of ethanol, and uniformly dispersing 252 g of magnesium stearate into the solution using a Polytron. Next, a 10 mg pulsed release-controlling coating of the following composition was coated onto each 59.7 mg intermediate coating-covered tablet using a pan coating machine, thus obtaining a controlled-release pharmaceutical composition containing 5 mg of rabeprazole sodium in a 69.7 mg tablet. The pulsed release-controlling coating was formed by spraying onto the intermediate coating-covered tablet an ethanol solution obtained by dissolving 120 g of Eudragit L100, 480 g of ethyl cellulose and 36 g of cetyl alcohol in 14.26 kg of ethanol, and adding 360 g of magnesium stearate, 90 g of talc and 54 g of titanium dioxide and uniformly dispersing using a Polytron.

**[Table 1]**

| Uncoated tablet | mg/Tablet | % W/W |
|---|---|---|
| Rabeprazole Sodium | 5.0 | 8.9 |
| D-mannitol | 33.6 | 60.0 |
| Crospovidone | 13.5 | 24.1 |
| Sodium hydroxide | 0.5 | 0.9 |
| Hydroxypropyl cellulose | 2.5 | 4.5 |
| Sodium stearyl fumarate | 0.9 | 1.6 |
| Subtotal | 56.0 | 100.0 |

| Intermediate coating | mg/Tablet | % W/W |
|---|---|---|
| Ethyl cellulose | 1.06 | 28.6 |
| Hydroxypropyl cellulose | 1.8 | 48.6 |
| Magnesium stearate | 0.84 | 22.7 |
| Subtotal | 3.7 | 100.0 |

| Pulsed release-controlling coating | mg/Tablet | % WNV |
|---|---|---|
| Eudragit L100 | 1.05 | 10.5 |
| Ethyl cellulose | 4.21 | 42.1 |
| Talc | 0.79 | 7.9 |
| Titanium dioxide | 0.47 | 4.7 |
| Cetyl alcohol | 0.32 | 3.2 |
| Magnesium stearate | 3.16 | 31.6 |
| Subtotal | 10.0 | 100.0 |

### Example 2

A 15 mg pulsed release-controlling coating of the following composition was coated using a pan coating machine onto intermediate coating-covered tablets each weighing 59.7 mg produced as in Example 1, thus obtaining a controlled-release pharmaceutical composition containing 5 mg of rabeprazole sodium in a 74.7 mg tablet.

**[Table 2]**

| Pulsed release-controlling coating | mg/Tablet | % W/W |
|---|---|---|
| Eudragit L100 | 1.58 | 10.5 |
| Ethyl cellulose | 6.32 | 42.1 |
| Talc | 1.18 | 7.9 |
| Titanium dioxide | 0.71 | 4.7 |
| Cetyl alcohol | 0.47 | 3.2 |
| Magnesium stearate | 4.74 | 31.6 |
| Subtotal | 15.0 | 100.0 |

### Example 3

A 20 mg pulsed release-controlling coating of the following composition was coated using a pan coating machine onto intermediate coating-covered tablets each weighing 59.7 mg produced as in Example 1, thus obtaining a controlled-release pharmaceutical composition containing 5 mg of rabeprazole sodium in a 79.7 mg tablet.

**[Table 3]**

| Pulsed release-controlling coating | mg/Tablet | % W/W |
|---|---|---|
| Eudragit L100 | 2.1 | 10.5 |
| Ethyl cellulose | 8.42 | 42.1 |
| Talc | 1.58 | 7.9 |
| Titanium dioxide | 0.94 | 4.7 |
| Cetyl alcohol | 0.64 | 3.2 |
| Magnesium stearate | 6.32 | 31.6 |
| Subtotal | 20.0 | 100.0 |

### Example 4

A 10 mg pulsed release-controlling coating of the following composition was coated using a pan coating machine onto intermediate coating-covered tablets each weighing 59.7 mg produced as in Example 1, thus obtaining a controlled-release pharmaceutical composition containing 5 mg of rabeprazole sodium in a 69.7 mg tablet.

The pulsed release-controlling coating was formed by spraying onto the intermediate coating-covered tablet an ethanol solution obtained by dissolving 180 g of Eudragit L100, 480 g of ethyl cellulose and 36 g of cetyl alcohol in 1500 g of ethanol, and adding 360 g of magnesium stearate, 90 g of talc and 54 g of titanium dioxide and uniformly dispersing using a Polytron.

**[Table 4]**

| Pulsed release-controlling coating | mg/Tablet | % W/W |
|---|---|---|
| Eudragit L100 | 1.50 | 15 |
| Ethyl cellulose | 4.00 | 40 |
| Talc | 0.75 | 7.5 |
| Titanium dioxide | 0.45 | 4.5 |
| Cetyl alcohol | 0.30 | 3 |
| Magnesium stearate | 3.00 | 30 |
| Subtotal | 10.0 | 100.0 |

### Example 5

A 15 mg pulsed release-controlling coating of the following composition was coated using a pan coating machine onto intermediate coating-covered tablets each weighing 59.7 mg using the same method as in Example 4, thus obtaining a controlled-release pharmaceutical composition containing 5 mg of rabeprazole sodium in a 74.7 mg tablet.

**[Table 5]**

| Pulsed release-controlling coating | mg/Tablet | % W/W |
|---|---|---|
| Eudragit L100 | 2.25 | 15 |
| Ethyl cellulose | 6.00 | 40 |
| Talc | 1.13 | 7.5 |
| Titanium dioxide | 0.68 | 4.5 |
| Cetyl alcohol | 0.45 | 3 |
| Magnesium stearate | 4.50 | 30 |
| Subtotal | 15.0 | 100.0 |

### Example 6

A 20.5 mg pulsed release-controlling coating of the following composition was coated using a pan coating machine onto intermediate coating-covered tablets each weighing 59.7 mg using the same method as in Example 4, thus obtaining a controlled-release pharmaceutical composition containing 5 mg of rabeprazole sodium in an 80.2 mg tablet.

**[Table 6]**

| Pulsed release-controlling coating | mg/Tablet | % W/W |
|---|---|---|
| Eudragit L100 | 3.08 | 15 |
| Ethyl cellulose | 8.20 | 40 |
| Talc | 1.54 | 7.5 |
| Titanium dioxide | 0.92 | 4.5 |
| Cetyl alcohol | 0.62 | 3 |
| Magnesium stearate | 6.15 | 30 |
| Subtotal | 20.5 | 100.0 |

### Example 7

Uncoated tablets of the following composition were produced, an intermediate coating was coated on, and then a release-controlling coating was coated on.

5.192 kg of mannitol, 3.96 kg of crospovidone and 0.33 kg of hydroxypropyl cellulose were added to and mixed with 2.2 kg of rabeprazole sodium, 4.4 kg of ethanol having 0.11 kg of sodium hydroxide dissolved therein was added, and granulation was carried out. The granules thus produced were dried using a tray dryer, and then passed through a 1.5 mm screen, and then 0.33 kg of crospovidone and 0.198 kg of sodium stearyl fumarate were added and mixed in, and tablet formation was carried out using a tablet machine, thus obtaining tablets (uncoated tablets) each weighing 56 mg and containing 10 mg of rabeprazole sodium. Next, 3 kg of the tablets were put into a coating pan, and an intermediate coating solution of the following composition was sprayed on, thus forming an intermediate coating in an amount of 3.7 mg per tablet. The intermediate coating solution was prepared by dissolving 191 g of ethyl cellulose and 324 g of hydroxypropyl cellulose in 9.58 kg of ethanol, and uniformly dispersing 151 g of magnesium stearate into the solution using a Polytron. Next, a 10 mg pulsed release-controlling coating of the following composition was coated onto each 59.7 mg intermediate coating-covered tablet using a pan coating machine, thus obtaining a controlled-release pharmaceutical composition containing 10 mg of rabeprazole sodium in a 69.7 mg tablet. The pulsed release-controlling coating was formed by spraying onto the intermediate coating-covered tablet an ethanol solution obtained by dissolving 134 g of Eudragit L100, 536 g of ethyl cellulose and 40 g of cetyl alcohol in 13.11 kg of ethanol, and adding 268 g of magnesium stearate, 101 g of talc and 60 g of titanium dioxide and uniformly dispersing using a Polytron.

**[Table 7]**

| Uncoated tablet | mg/Tablet | % W/W |
|---|---|---|
| Rabeprazole Sodium | 10.0 | 17.9 |
| D-mannitol | 23.6 | 42.1 |
| Crospovidone | 18.0 | 32.1 |
| Sodium hydroxide | 0.5 | 0.9 |
| Hydroxypropyl cellulose | 1.5 | 2.7 |
| Sodium stearyl fumarate | 0.9 | 1.6 |
| Subtotal | 56.0 | 100.0 |

| Intermediate coating | mg/Tablet | % W/W |
|---|---|---|
| Ethyl cellulose | 1.06 | 28.6 |
| Hydroxypropyl cellulose | 1.8 | 48.6 |
| Magnesium stearate | 0.84 | 22.7 |
| Subtotal | 3.7 | 100.0 |

| Pulsed release-controlling coating | mg/Tablet | % W/W |
|---|---|---|
| Eudragit L100 | 0.94 | 11.8 |
| Ethyl cellulose | 3.76 | 47.1 |
| Talc | 0.71 | 8.8 |
| Titanium dioxide | 0.42 | 5.3 |
| Cetyl alcohol | 0.28 | 3.5 |
| Magnesium stearate | 1.88 | 23.5 |
| Subtotal | 8.0 | 100.0 |

### Example 8

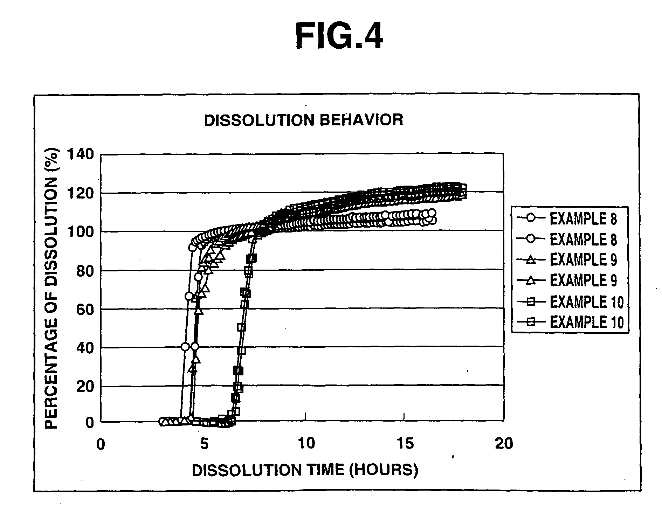

An 8 mg pulsed release-controlling coating of the following composition was coated using a pan coating machine onto intermediate coating-covered tablets each weighing 59.7 mg using the same method as in Example 7, thus obtaining a controlled-release pharmaceutical composition containing 10 mg of rabeprazole sodium in a 67.7 mg tablet. Dissolution test results for the controlled-release pharmaceutical composition according to the method of dissolution test (1) described earlier are shown in FIG. 4.

**[Table 8]**

| Pulsed release-controlling coating | mg/Tablet | % W/W |
|---|---|---|
| Eudragit L100 | 0.86 | 10.7 |
| Ethyl cellulose | 3.42 | 42.8 |
| Talc | 0.64 | 8.0 |
| Titanium dioxide | 0.39 | 4.8 |
| Cetyl alcohol | 0.13 | 1.6 |
| Magnesium stearate | 2.57 | 32.1 |
| Subtotal | 8.0 | 100.0 |

### Example 9

Uncoated tablets of the following composition were produced, an intermediate coating was coated on, and then a release-controlling coating was coated on.

3.0 kg of mannitol, 5.0 kg of magnesium oxide, 0.6 kg of hydroxypropyl cellulose and 0.9 kg of low-substituted hydroxypropyl cellulose were added to and mixed with 1.0 kg of rabeprazole sodium, 3.4 L of ethanol was added, and granulation was carried out. The granules thus produced were dried using a tray dryer, and then passed through a 1.5 mm screen, and then 0.58 kg of low-substituted hydroxypropyl cellulose and 0.12 kg of magnesium stearate were added and mixed in, and tablet formation was carried out using a tablet machine, thus obtaining tablets (uncoated tablets) each weighing 56 mg and containing 5 mg of rabeprazole sodium. Intermediate coating-covered tablets were then produced as in Example 1, thus obtaining a pharmaceutical with a weight per tablet of 59.7 mg. A 6 mg pulsed release-controlling coating of the following composition was then coated on using a pan coating machine, thus obtaining a controlled-release pharmaceutical composition containing 5 mg of rabeprazole sodium in a 65.7 mg tablet.

Dissolution test results for the controlled-release pharmaceutical composition according to the method of dissolution test (1) described earlier are shown in FIG. 4.

**[Table 9]**

| Uncoated tablet | mg/Tablet | % W/W |
|---|---|---|
| Rabeprazole Sodium | 5.0 | 8.9 |
| D-mannitol | 15.0 | 26.8 |
| Magnesium oxide | 25.0 | 44.6 |
| Low-substituted hydroxypropyl cellulose | 7.4 | 13.2 |
| Hydroxypropyl cellulose | 3.0 | 5.4 |
| Magnesium stearate | 0.6 | 1.1 |
| Subtotal | 56.0 | 100.0 |

| Intermediate coating | mg/Tablet | % W/W |
|---|---|---|
| Ethyl cellulose | 1.06 | 28.6 |
| Hydroxypropyl cellulose | 1.8 | 48.6 |
| Magnesium stearate | 0.84 | 22.7 |
| Subtotal | 3.7 | 100.0 |

| Pulsed release-controlling coating | mg/Tablet | % W/W |
|---|---|---|
| Eudragit L100 | 0.77 | 12.9 |
| Ethyl cellulose | 3.09 | 51.5 |
| Talc | 0.59 | 9.9 |
| Titanium dioxide | 036 | 5.9 |
| Cetyl alcohol | 0.59 | 9.9 |
| Magnesium stearate | 0.59 | 9.9 |
| Subtotal | 6.0 | 100.0 |

### Example 10

A 15 mg pulsed release-controlling coating of the following composition was coated using a pan coating machine onto intermediate coating-covered tablets each weighing 59.7 mg produced as in Example 1, thus obtaining a controlled-release pharmaceutical composition containing 5 mg of rabeprazole sodium in a 74.7 mg tablet. Dissolution test results for the controlled-release pharmaceutical composition according to the method of dissolution test (1) described earlier are shown in FIG. 4.

**[Table 10]**

| Pulsed release-controlling coating | mg/Tablet | % W/W |
|---|---|---|
| Eudragit L100 | 1.67 | 11.1 |
| Ethyl cellulose | 6.97 | 46.5 |
| Titanium dioxide | 0.76 | 5.1 |
| Cetyl alcohol | 0.45 | 3.0 |
| Magnesium stearate | 5.15 | 34.3 |
| Subtotal | 15 | 100.0 |

### Example 11

Uncoated tablets of the following composition were produced, an intermediate coating was coated on, and then a release-controlling coating was coated on.

4.92 kg of mannitol and 3 kg of crospovidone were added to and mixed with 2 kg of rabeprazole sodium, 4 kg of ethanol having 0.1 kg of sodium hydroxide dissolved therein was added, and granulation was carried out. The granules thus produced were dried using a tray dryer, and then passed through a 1 mm screen, and then 0.3 kg of crospovidone and 0.18 kg of sodium stearyl fumarate were added and mixed in, and tablet formation was carried out using a tablet machine, thus preparing tablets (uncoated tablets) each weighing 52.5 mg and containing 10 mg of rabeprazole sodium. Next, the uncoated tablets were made to flow in a fluidized bed coating apparatus, and an intermediate coating solution obtained by dissolving 651 g of hydroxypropyl cellulose in 12.52 kg of ethanol and uniformly dispersing 219 g of calcium stearate into the solution was sprayed on, thus forming an intermediate coating in an amount of 2.9 mg per tablet, and hence preparing intermediate coating-covered tablets each weighing 55.4 mg and containing 10 mg of rabeprazole sodium. Moreover, separately, an ethanol solution obtained by dissolving 1980 g of Eudragit L100, 495 g of ethyl cellulose and 446 g of triethyl citrate in 55 kg of ethanol, and adding 1485 g of calcium stearate, 372 g of talc and 223 g of titanium dioxide and uniformly dispersing was prepared, and was sprayed onto the intermediate coating-covered tablets flowing in the fluidized bed, thus forming an 8 mg pulsed release-controlling coating, and hence producing a controlled-release pharmaceutical composition containing 10 mg of rabeprazole sodium in a 63.4 mg tablet.

**[Table 11]**

| Uncoated tablet | mg/Tablet | % W/W |
|---|---|---|
| Rabeprazole Sodium | 10.0 | 19.0 |
| D-mannitol | 24.6 | 46.9 |
| Sodium hydroxide | 0.5 | 1.0 |
| Crospovidone | 16.5 | 31.4 |
| Sodium stearyl fumarate | 0.9 | 1.7 |
| Subtotal | 52.5 | 100.0 |

| Intermediate coating | mg/Tablet | % W/W |
|---|---|---|
| Hydroxypropyl cellulose | 2.17 | 74.8 |
| Calcium stearate | 0.73 | 25.2 |
| Subtotal | 2.9 | 100.0 |

| Pulsed release-controlling coating | mg/Tablet | % W/W |
|---|---|---|
| Eudragit L100 | 3.17 | 39.6 |
| Ethyl cellulose | 0.79 | 9.9 |
| Talc | 0.59 | 7.4 |
| Titanium dioxide | 0.36 | 4.5 |
| Triethyl citrate | 0.71 | 8.9 |
| Calcium stearate | 2.38 | 29.7 |
| Subtotal | 8.0 | 100.0 |

### Example 12

Uncoated tablets of the following composition were produced, an intermediate coating was coated on, and then a release-controlling coating was coated on.

4.92 kg of mannitol and 3 kg of crospovidone were added to and mixed with 2 kg of rabeprazole sodium, 4 kg of ethanol having 0.1 kg of sodium hydroxide dissolved therein was added, and granulation was carried out. The granules thus produced were dried using a tray dryer, and then passed through a 1 mm screen, and then 0.3 kg of crospovidone and 0.18 kg of sodium stearyl fumarate were added and mixed in, and tablet formation was carried out using a tablet machine, thus preparing tablets (uncoated tablets) each weighing 52.5 mg and containing 10 mg of rabeprazole sodium. Next, the uncoated tablets were made to flow in a fluidized bed coating apparatus, and an intermediate coating solution obtained by dissolving 651 g of hydroxypropyl cellulose in 12.52 kg of ethanol and uniformly dispersing 219 g of calcium stearate into the solution was sprayed on, thus forming an intermediate coating in an amount of 2.9 mg per tablet, and hence preparing intermediate coating-covered tablets each weighing 55.4 mg and containing 10 mg of rabeprazole sodium. Moreover, separately, an ethanol solution obtained by dissolving 2200 g of Eudragit L100, 275 g of ethyl cellulose and 446 g of triethyl citrate in 55 kg of ethanol, and adding 1485 g of calcium stearate, 372 g of talc and 223 g of titanium dioxide and uniformly dispersing was prepared, and was sprayed onto the intermediate coating-covered tablets flowing in the fluidized bed, thus forming an 8 mg pulsed release-controlling coating, and hence producing a controlled-release pharmaceutical composition containing 10 mg of rabeprazole sodium in a 63.4 mg tablet.

**[Table 12]**

| Uncoated tablet | mg/Tablet | % W/W |
|---|---|---|
| Rabeprazole Sodium | 10.0 | 19.0 |
| D-mannitol | 24.6 | 46.9 |
| Sodium hydroxide | 0.5 | 1.0 |
| Crospovidone | 16.5 | 31.4 |
| Sodium stearyl fumarate | 0.9 | 1.7 |
| Subtotal | 52.5 | 100.0 |

| Intermediate coating | mg/Tablet | % W/W |
|---|---|---|
| Hydroxypropyl cellulose | 2.17 | 74.8 |
| Calcium stearate | 0.73 | 25.2 |
| Subtotal | 2.9 | 100.0 |

| Pulsed release-controlling coating | mg/Tablet | % W/W |
|---|---|---|
| Eudragit L100 | 3.52 | 44.0 |
| Ethyl cellulose | 0.44 | 5.5 |
| Talc | 0.59 | 7.4 |
| Titanium dioxide | 0.36 | 4.5 |
| Triethyl citrate | 0.71 | 8.9 |
| Calcium stearate | 2.38 | 29.7 |
| Subtotal | 8.0 | 100.0 |

### Example 13

6, 10 or 14 mg pulsed release-controlling coatings of the following composition were coated using a pan coating machine onto intermediate coating-covered tablets each weighing 55.4 mg using the same method as in Example 11, thus obtaining controlled-release pharmaceutical compositions each containing 10 mg of rabeprazole sodium in a tablet.

**[Table 13]**

| Pulsed release-controlling coating | % W/W |
|---|---|
| Eudragit L100 | 42.5 |
| Ethyl cellulose | 7.0 |
| Talc | 7.4 |
| Titanium dioxide | 4.5 |
| Triethyl citrate | 8.9 |
| Calcium stearate | 29.7 |
| Subtotal | 100.0 |

### Example 14

6, 10 or 14 mg pulsed release-controlling coatings of the following composition were coated using a pan coating machine onto intermediate coating-covered tablets each weighing 55.4 mg using the same method as in Example 11, thus obtaining controlled-release pharmaceutical compositions each containing 10 mg of rabeprazole sodium in a tablet.

**[Table 14]**

| Pulsed release-controlling coating | % W/W |
|---|---|
| Eudragit L100 | 42.5 |
| Ethyl cellulose | 7.0 |
| Talc | 7.4 |
| Titanium dioxide | 4.5 |
| Cetyl alcohol | 8.9 |
| Calcium stearate | 29.7 |
| Subtotal | 100.0 |

### Example 15

6, 10 or 14 mg pulsed release-controlling coatings of the following composition were coated using a pan coating machine onto intermediate coating-covered tablets each weighing 55.4 mg using the same method as in Example 11, thus obtaining controlled-release pharmaceutical compositions each containing 10 mg of rabeprazole sodium in a tablet.

**[Table 15]**

| Pulsed release-controlling coating | % W/W |
|---|---|
| Eudragit L100 | 42.5 |
| Ethyl cellulose | 7.0 |
| Talc | 7.4 |
| Titanium dioxide | 4.5 |
| Glycerol fatty acid ester | 8.9 |
| Calcium stearate | 29.7 |
| Subtotal | 100.0 |

### Example 16

Uncoated tablets of the following composition were produced, an intermediate coating was coated on, and then an enteric coating was coated on.

5.192 kg of mannitol, 3.96 kg of crospovidone and 0.33 kg of hydroxypropyl cellulose were added to and mixed with 2.2 kg of rabeprazole sodium, 4.4 kg of ethanol having 0.11 kg of sodium hydroxide dissolved therein was added, and granulation was carried out. The granules thus produced were dried using a tray dryer, and then passed through a 1.5 mm screen, and then 0.33 kg of crospovidone and 0.198 kg of sodium stearyl fumarate were added and mixed in, and tablet formation was carried out using a tablet machine, thus preparing tablets (uncoated tablets) each weighing 56 mg and containing 10 mg of rabeprazole sodium. Next, the uncoated tablets were made to flow in a fluidized bed coating apparatus, and an intermediate coating solution obtained by dissolving 191 g of ethyl cellulose and 324 g of hydroxypropyl cellulose in 9.58 kg of ethanol and uniformly dispersing 151 g of magnesium stearate into the solution was sprayed on, thus forming an intermediate coating in an amount of 3.7 mg per tablet, and hence preparing intermediate coating-covered tablets each weighing 59.7 mg and containing 10 mg of rabeprazole sodium. Moreover, separately, an enteric coating solution was prepared by dissolving 1726 g of hydroxypropyl methyl cellulose phthalate and 172 g of glycerol fatty acid ester in 20.8 kg of 80% ethanol and adding a suspension obtained by uniformly dispersing 163 g of talc, 10 g of yellow iron oxide and 87 g of titanium dioxide in 5.2 kg of an 80% ethanol solution, and the enteric coating solution was sprayed onto the intermediate coating-covered tablets flowing in the fluidized bed coating apparatus, thus forming an 8.3 mg enteric coating, and hence producing an enteric pharmaceutical composition containing 10 mg of rabeprazole sodium in a 67.2 mg tablet.

**[Table 16]**

| Uncoated tablet | mg/Tablet | % W/W |
|---|---|---|
| Rabeprazole Sodium | 10.0 | 17.9 |
| D-mannitol | 23.6 | 42.1 |
| Crospovidone | 18.0 | 32.1 |
| Sodium hydroxide | 0.5 | 0.9 |
| Hydroxypropyl cellulose | 1.5 | 2.7 |
| Sodium stearyl fumarate | 0.9 | 1.6 |
| Subtotal | 56.0 | 100.0 |

| Intermediate coating | mg/Tablet | % W/W |
|---|---|---|
| Ethyl cellulose | 1.06 | 28.6 |
| Hydroxypropyl cellulose | 1.8 | 48.6 |
| Magnesium stearate | 0.84 | 22.7 |
| Subtotal | 3.7 | 100.0 |

| Enteric coating | mg/Tablet | % W/W |
|---|---|---|
| Hydroxypropyl methyl cellulose phthalate | 6.64 | 80.0 |
| Glycerol fatty acid ester | 0.66 | 8.0 |
| Talc | 0.63 | 7.5 |
| Titanium dioxide | 0.33 | 4.0 |
| Yellow iron oxide | 0.04 | 0.5 |
| Subtotal | 8.0 | 100.0 |

To show the remarkable effects of the controlled-release pharmaceutical compositions according to the above examples, controls will now be described.

### Control 1

A 5 mg coating of the following composition (not containing magnesium stearate) was coated using a pan coating machine onto intermediate coating-covered tablets each weighing 59.7 mg produced as in Example 1, thus obtaining a pharmaceutical composition containing 5 mg of rabeprazole sodium in a 64.7 mg tablet.

**[Table 17]**

| Coating | mg/Tablet | % W/W |
|---|---|---|
| Eudragit L100 | 2.00 | 40 |
| Ethyl cellulose | 2.00 | 40 |
| Talc | 0.38 | 7.5 |
| Titanium dioxide | 0.23 | 4.5 |
| Cetyl alcohol | 0.40 | 8 |
| Subtotal | 5.0 | 100.0 |

### Control 2

A 10 mg coating of the following composition (not containing magnesium stearate) was coated using a pan coating machine onto intermediate coating-covered tablets each weighing 59.7 mg produced as in Example 1, thus obtaining a pharmaceutical composition containing 5 mg of rabeprazole sodium in a 69.7 mg tablet.

**[Table 18]**

| Coating | mg/Tablet | % W/W |
|---|---|---|
| Eudragit L100 | 4.0 | 40 |
| Ethyl cellulose | 4.0 | 40 |
| Talc | 0.76 | 7.5 |
| Titanium dioxide | 0.46 | 4.5 |
| Cetyl alcohol | 0.8 | 8 |
| Subtotal | 10.0 | 100.0 |

### Control 3

A 5 mg coating of the following composition (not containing magnesium stearate) was coated using a pan coating machine onto intermediate coating-covered tablets each weighing 59.7 mg produced as in Example 1, thus obtaining a pharmaceutical composition containing 5 mg of rabeprazole sodium in a 64.7 mg tablet.

**[Table 19]**

| Coating | mg/Tablet | % W/W |
|---|---|---|
| Eudragit L100 | 0.77 | 15.4 |
| Ethyl cellulose | 3.08 | 61.5 |
| Talc | 0.58 | 11.5 |
| Titanium dioxide | 0.35 | 6.9 |
| Cetyl alcohol | 0.23 | 4.6 |
| Subtotal | 5.0 | 100.0 |

### Control 4

A 15 mg coating of the following composition (not containing magnesium stearate) was coated using a pan coating machine onto intermediate coating-covered tablets each weighing 59.7 mg produced as in Example 1, thus obtaining a pharmaceutical composition containing 5 mg of rabeprazole sodium in a 74.7 mg tablet.

**[Table 20]**

| Coating | mg/Tablet | % W/W |
|---|---|---|
| Eudragit L100 | 6.0 | 42.1 |
| Ethyl cellulose | 6.0 | 42.1 |
| Talc | 1.125 | 7.9 |
| Titanium dioxide | 0.675 | 4.7 |
| Cetyl alcohol | 1.20 | 3.2 |
| Subtotal | 15.0 | 100.0 |

### Control 5

A 20 mg coating of the following composition (not containing magnesium stearate) was coated using a pan coating machine onto intermediate coating-covered tablets each weighing 59.7 mg produced as in Example 1, thus obtaining a pharmaceutical composition containing 5 mg of rabeprazole sodium in a 79.7 mg tablet.

**[Table 21]**

| Coating | mg/Tabtet | % W/W |
|---|---|---|
| Eudragit L100 | 8.0 | 42.1 |
| Ethyl cellulose | 8.0 | 42.1 |
| Talc | 1.5 | 7.9 |
| Titanium dioxide | 0.9 | 4.7 |
| Cetyl alcohol | 1.6 | 3.2 |
| Subtotal | 20.0 | 100.0 |

### Control 6

A 25 mg coating of the following composition (not containing magnesium stearate) was coated using a pan coating machine onto intermediate coating-covered tablets each weighing 59.7 mg produced as in Example 1, thus obtaining a pharmaceutical composition containing 5 mg of rabeprazole sodium in an 84.7 mg tablet.

**[Table 22]**

| Coating | mg/Tablet | % W/W |
|---|---|---|
| Eudragit L100 | 10.0 | 42.1 |
| Ethyl cellulose | 10.0 | 42.1 |
| Talc | 1.875 | 7.9 |
| Titanium dioxide | 1.125 | 4.7 |
| Cetyl alcohol | 2.0 | 3.2 |
| Subtotal | 25.0 | 100.0 |

### Control 7

A 30 mg coating of the following composition (not containing magnesium stearate) was coated using a pan coating machine onto intermediate coating-covered tablets each weighing 59.7 mg produced as in Example 1, thus obtaining a pharmaceutical composition containing 5 mg of rabeprazole sodium in an 89.7 mg tablet.

**[Table 23]**

| Coating | mg/Tablet | % W/W |
|---|---|---|
| Eudragit L100 | 12.0 | 42.1 |
| Ethyl cellulose | 12.0 | 42.1 |
| Talc | 2.25 | 7.9 |
| Titanium dioxide | 1.35 | 4.7 |
| Cetyl alcohol | 2.4 | 3.2 |
| Subtotal | 30.0 | 100.0 |

### Industrial Applicability

According to the present invention, in the case of a controlled-release pharmaceutical composition, particularly a pulsed-release pharmaceutical composition, containing an acid-unstable physiologically active substance, a pharmaceutical composition having little variation in dissolution lag time and percentage of dissolution over time, and high reliability of dissolution characteristics can be realized. Furthermore, a capsule preparation obtained by filling an enteric pharmaceutical composition and the controlled-release pharmaceutical composition according to the present invention into a capsule enables design of a pharmaceutical composition having an increased medical benefit duration.

## Claims

1. A controlled-release pharmaceutical composition, comprising:
1) a core containing an acid-unstable physiologically active substance and a disintegrant; and
2) a release-controlling coating which covers the core, and which contains a water-insoluble polymer, an enteric polymer and a hydrophobic wax.

2. The controlled-release pharmaceutical composition according to claim 1, wherein the release-controlling coating further comprises a plasticizer.

3. The controlled-release pharmaceutical composition according to claim 1 or 2, wherein the core further comprises an alkaline additive.

4. The controlled-release pharmaceutical composition according to any one of claims 1 through 3, further comprising an inert intermediate coating between the core and the release-controlling coating.

5. The controlled-release pharmaceutical composition according to any one of claims 1 through 4, wherein the controlled-release pharmaceutical composition is a pulsed-release pharmaceutical composition.

6. The controlled-release pharmaceutical composition according to any one of claims 1 through 5, wherein the disintegrant is at least one selected from the group consisting of crospovidone, low-substituted hydroxypropyl cellulose, croscarmellose sodium, and carmellose calcium.

7. The controlled-release pharmaceutical composition according to any one of claims 1 through 6, wherein the water-insoluble polymer is at least one selected from the group consisting of ethyl cellulose, an aminoalkyl methacrylate copolymer RS (Eudragit RS), and shellac.

8. The controlled-release pharmaceutical composition according to any one of claims 1 through 7, wherein the enteric polymer is at least one selected from the group consisting of hydroxypropyl methyl cellulose phthalate, hydroxypropyl methyl cellulose acetate succinate, a methacrylic acid-methyl methacrylate copolymer (Eudragit L, Eudragit S), and a methacrylic acid-ethyl acrylate copolymer (Eudragit LD).

9. The controlled-release pharmaceutical composition according to any one of claims 1 through 8, wherein the hydrophobic wax is at least one selected from the group consisting of magnesium stearate, calcium stearate, stearic acid, carnauba wax, and a hydrogenated oil.

10. The controlled-release pharmaceutical composition according to any one of claims 1 through 9, wherein the water-insoluble polymer is ethyl cellulose, the enteric polymer is a methacrylic acid-methyl methacrylate copolymer (Eudragit L, Eudragit S), and the hydrophobic wax is magnesium stearate or calcium stearate.

11. The controlled-release pharmaceutical composition according to any one of claims 2 through 10, wherein the plasticizer is at least one selected from the group consisting of triethyl citrate, cetyl alcohol, glycerol fatty acid ester, and propylene glycol.

12. The controlled-release pharmaceutical composition according to any one of claims 1 through 11, wherein a total amount of the water-insoluble polymer and the enteric polymer in the release-controlling coating is 40 to 90 wt%, based on the weight of the release-controlling coating.

13. The controlled-release pharmaceutical composition according to any one of claims 1 through 12, wherein an amount of the hydrophobic wax in the release-controlling coating is 10 to 60 wt%, based on the weight of the release-controlling coating.

14. The controlled-release pharmaceutical composition according to any one of claims 1 through 13, wherein an amount of the water-insoluble polymer in the release-controlling coating is 3.0 to 95 wt%, based on the total amount of the water-insoluble polymer and the enteric polymer in the release-controlling coating.

15. The controlled-release pharmaceutical composition according to any one of claims 2 through 14, wherein an amount of the plasticizer in the release-controlling coating is 0.1 to 20 wt%, based on the weight of the release-controlling coating.

16. The controlled-release pharmaceutical composition according to any one of claims 1 through 15, wherein the acid-unstable physiologically active substance is a benzimidazole-based compound or a physiologically acceptable salt thereof.

17. The controlled-release pharmaceutical composition according to claim 16, wherein the benzimidazole-based compound or physiologically acceptable salt thereof is rabeprazole, omeprazole, pantoprazole, lansoprazole or esomeprazole, or a physiologically acceptable salt thereof.

18. The controlled-release pharmaceutical composition according to claim 16 or 17, wherein the benzimidazole-based compound or physiologically acceptable salt thereof is rabeprazole sodium.

19. The controlled-release pharmaceutical composition according to any one of claims 3 through 18, wherein the alkaline additive is at least one selected from the group consisting of sodium hydroxide, potassium hydroxide, magnesium oxide, calcium oxide, magnesium hydroxide, and calcium hydroxide.

20. The controlled-release pharmaceutical composition according to any one of claims 1 through 19, wherein the controlled-release pharmaceutical composition is a tablet, a granular preparation, or a fine granular preparation.

21. A capsule preparation, comprising:
the controlled-release pharmaceutical composition according to any one of claims 1 through 20; and
an enteric pharmaceutical composition in which a core containing an acid-unstable physiologically active substance is covered with an enteric coating.

22. A pharmaceutical composition package contained in a packaging container, comprising:
the controlled-release pharmaceutical composition according to any one of claims 1 through 20; and
an enteric pharmaceutical composition in which a core containing an acid-unstable physiologically active substance is covered with an enteric coating,
wherein both of the composition are present in the same packaging container.

23. A pharmaceutical composition package contained in a packaging container, comprising:
the capsule preparation according to claim 21.

24. The pharmaceutical composition package according to claim 22 or 23, wherein the packaging is sachet or blister packaging.

25. A method for producing a controlled-release pharmaceutical composition comprising:
forming a release-controlling coating by spraying a solution containing a mixture of a water-insoluble polymer, an enteric polymer and a hydrophobic wax onto a core containing an acid-unstable physiologically active substance and a disintegrant to form a coating covering the core.

26. The method for producing a controlled-release pharmaceutical composition according to claim 25, wherein the release-controlling coating further comprises a plasticizer.

27. The method for producing a controlled-release pharmaceutical composition according to claim 25 or 26, wherein the core further comprises an alkaline additive.

28. The method for producing a controlled-release pharmaceutical composition according to any one of claims 25 through 27, further comprising forming an inert intermediate coating between the core and the release-controlling coating.

29. The method for producing a controlled-release pharmaceutical composition according to any one of claims 25 through 28, wherein the controlled-release pharmaceutical composition is a pulsed-release pharmaceutical composition.

30. A method of controlling release to reduce variation in a dissolution lag time, comprising: covering a core containing an acid-unstable physiologically active substance and a disintegrant with a release-controlling coating containing a water-insoluble polymer, an enteric polymer and a hydrophobic wax.
